# EUROPEAN PATENT SPECIFICATION

(11) **EP 4 308 930 B1**
(45) Date of publication and mention of the grant of the patent: **20.05.2026**
(21) Application number: 22710160.7
(22) Date of filing: 04.03.2022
(51) Int. Cl.: G01N 33/543, C03C 11/00

(54) **COMPOSITIONS AND METHODS OF DETECTING ANALYTES**
ZUSAMMENSETZUNGEN UND VERFAHREN ZUM NACHWEIS VON ANALYTEN
COMPOSITIONS ET PROCÉDÉS DE DÉTECTION D'ANALYTES

(30) Priority: 19.03.2021 US 202163163082 P
(43) Date of publication of application: 24.01.2024
(73) Proprietor: Neogen Food Safety US HoldCo Corporation, Lansing, MI 48912 (US)
(72) Inventor: CHANDRAPATI, Sailaja, Saint Paul, Minnesota 55133-3427 (US); HENNA, Phillip H., Saint Paul, Minnesota 55133-3427 (US); SGOLASTRA, Federica, Saint Paul, Minnesota 55133-3427 (US); SCHULTZ, Kimberly C.M., Saint Paul, Minnesota 55133-3427 (US)
(74) Representative: Ter Meer Steinmeister & Partner
(86) International application number: PCT/IB2022/051935
(87) International publication number: WO 2022/195394

(56) References cited:
- EP-A1- 1 833 767
- WO-A2-2016/090359
- CN-A- 107 602 919
- DONG ZIYE ET AL: "Cell Isolation and Recovery Using Hollow Glass Microspheres Coated with Nanolayered Films for Applications in Resource-Limited Settings", ACS APPLIED MATERIALS & INTERFACES, WASHINGTON, DC : ACS, 2009-, US, vol. 9, no. 18, 10 May 2017 (2017-05-10), pages 15265 - 15273, XP009501640, ISSN: 1944-8252, DOI: 10.1021/ACSAMI.7B02197

## Description

### BACKGROUND

Wang et al. (Analytica Chimca Acta 912 (2016) 10-23) disclose nanoparticle-based immunosensors and immunoassays for aflatoxins. Among the sensors disclosed are fluorescence assays, and chemiluminescence assays, and light-scattering assays. Becheva et al. (International Journal of Food Science and Technology 2018) disclose a rapid immunofluorescence assay for staphylococcal enterotoxin A using magnetic nanoparticles. The assay uses antibody immobilized on magnetic nanoparticles. During the assay, magnetic nanoparticles are removed from the liquid by way of a magnet and the fluorescent signal of the supernatant is measured.

Lateral flow assays are known, for example, as self-administered pregnancy tests. These assays do not require special analytical equipment. Quantitation of analytes by ELISA (Enzyme Linked Immunosorbent Assay) is known.

Polymers for extended release are discussed in a variety of references, such as Paolini, M.S., Fenton, O.S., Bhattacharya, C. et al. Polymers for extended-release administration. Biomed Microdevices 21, 45 (2019) and Polymers Kamaly, N., Yameen, B., Wu, J. et al. Degradable controlled-release polymers and polymeric nanoparticles: mechanisms of controlling drug release. Chem. Rev. 2016, 116, 4, 2602-2663.

WO 2016/090359 A2 relates to compositions and methods for glass composites being suitable for tissue augmentation, biomedical, and cosmetic applications. The glass microsphere component of the composites are biologically inert, non-reactive and act as a nearly permanent tissue filler. The compositions can be used for soft or hard tissue augmentation as well as delivery of cargos on demand.

### SUMMARY OF THE DISCLOSURE

One aspect of the invention is a composition comprising:
a film disposed on a substrate, the film comprising
   a first polymer matrix material; and
   a plurality of first hollow glass bubbles embedded in the first polymer matrix material;
wherein the first hollow glass bubbles have a density of less than 0.60 gram/mL, a span of less than 1.0, and a plurality of first affinity groups that are covalently attached to the first hollow glass bubbles.

Another aspect of the invention is a method of detecting an analyte, comprising:
providing a composition according to the invention;
contacting a sample containing first free target analyte molecules with the composition;
contacting a liquid, optionally an aqueous liquid, with the composition;
allowing the first free target analyte molecules to bind to the first affinity groups or to the first detector compound molecules;
allowing the plurality of first hollow glass bubbles to float to an upper concentrated portion of the liquid; and
measuring an amount of the first detector compound molecules in a second portion of the liquid that is below the upper concentrated portion, optionally without removing the plurality of first hollow glass bubbles from the liquid.

Preferred embodiments are defined by the dependent claims.

### BRIEF DESCRIPTION OF THE DRAWINGS

Figure 1 is a representation of a method of the present disclosure using a bound antibody elution method with detector compound molecules initially complexed with bound antibodies to detect free (i.e., unbound) target analyte molecules by measuring displaced (i.e., eluted) detector compound molecules.
Figure 2 is a representation of a method of the present disclosure using a bound antibody depletion method with free detector compound molecules and bound antibodies to detect free target analyte molecules by measuring (remaining free) detector compound molecules.
Figure 3 is a representation of a method of the present disclosure using a bound target elution method with labelled antibodies (detector compound molecules that include a detector
group (i.e., the label) bonded with a complementary group (i.e., the antibody)), which are complexed with bound target molecules, to detect free target analytes by measuring labelled antibodies displaced (i.e., eluted) by the initially free target analyte molecules.
Figure 4 is a representation of a method of the present disclosure using a bound target depletion method with free initially labelled antibodies (detector compound molecules that include a detector group (i.e., the label) bonded with a complementary group (i.e., the antibody)), to detect free target analytes by measuring labelled antibodies complexed with initially free target analyte molecules.
Figures 5A and 5B are size distribution charts of glass bubbles before and after fractionation.
Figures 6 and 7 are graphs of the percent increase in fluorescence (at two different wavelengths) over a control sample for solutions containing 10 ppb, 5 ppb, or 1 ppb of either cortisol or fumonisin, or mixtures containing 10 ppb, 5 ppb, or 1 ppb of each.
Figure 8 is a graph of the percent increase in signal over a control sample for 50 ppb of either cortisol, fumonisin, aflatoxin Bl, or a mixture of all three at different excitation and emission wavelengths.
Figure 9 is a graph of the percent increase in signal over a control sample for 5 ppb of either cortisol, fumonisin, aflatoxin Bl, or a mixture of all three at different excitation and emission wavelengths.
Figure 10 is a graph of fluorescence from solutions containing fumonisin in either grain extract or Phosphate Buffered Saline (PBS).
Figure 11 is a graph of fluorescence intensity in the detection of aflatoxin B 1 by the depletion method.

### DETAILED DESCRIPTION OF ILLUSTRATIVE EMBODIMENTS

The term "affinity group" refers to a covalently attached group that is capable of specifically binding another molecule. The affinity group may be further complexed with, or bonded to, a detector compound, that can be detected, e.g., by fluorescence, luminescence, or absorption at a particular wavelength. Herein, the affinity group is distinct from the detector compound or detector group.

The term, "antibody" refers to polypeptide molecules that contain regions that are capable of binding, and particularly specifically binding, a target analyte; thus the term includes both complete antibodies and in some cases antibody fragments.

The terms "specifically binding" and "specific binding" mean that an affinity group complexes with another molecule (i.e., its complementary molecule) with greater affinity than it complexes with other molecules under the assay conditions. For example, binding between an antibody and a complementary target molecule is a specific binding interaction. In particular cases "specifically binding" may mean that an affinity group complexes with a complementary molecule with at least a 106-fold greater affinity, at least a 107-fold greater affinity, at least a 108-fold greater affinity, or at least a 109-fold greater affinity than it complexes with molecules unrelated to the target molecule; in this context, a molecule is unrelated to the target molecule if it lacks the portions of the chemical structure of the target molecule that interact with the affinity group.

The term "span" is the particle size distribution of a sample of particles, which can be expressed by the following formula: Span = (90P-10P)/50P, wherein: 90P is the size for which 90 percent (%) of the particles in the distribution are smaller (referred to as the 90th percentile size); 10P is the size for which only 10% of the particles in the distribution are smaller (referred to as the 10th percentile size); 50P is the size for which 50 percent of the particles in the distribution are smaller (referred to as the 50th percentile size).

In this application, terms such as "a," "an," and "the" are not intended to refer to only a singular entity but include the general class of which a specific example may be used for illustration. The terms "a," "an," and "the" are used interchangeably with the phrases "at least one" and "one or more." The phrases "at least one of' and "comprises at least one of' followed by a list refers to any one of the items in the list and any combination of two or more items in the list.

Terms such as "common," "commonly," "often," "frequent," and "frequently" are used to refer to features that are typically employed in the invention, but unless otherwise indicated are not meant to imply that the features so described were known or common before this disclosure.

Magnetic beads or nanoparticles have been used as a solid support component for heterogeneous assays that detect target analytes. A typical prior-art assay using magnetic beads or nanoparticles is a competitive assay where the beads are modified with molecule, such as an antibody, that can bind to the target analyte. An amount, usually a known amount, of target analyte bound to a detection molecule, such as a luminescent dye, is added to an aqueous liquid containing the metal beads or nanoparticles. The assay is then a competitive binding assay where the detector-bound luminescent analyte and the unbound analyte from the sample compete to bind with the magnetic bead or nanoparticle bound antibody, and the luminescence of the liquid is measured. A magnet is then applied to the aqueous liquid to remove the magnetic beads or nanoparticles, along with whatever is bound to them, from the liquid. The fluorescence of the supernatant is then measured and change in luminescence can be related to the concentration of target analyte in the sample.

There are problems with the use of magnetic beads or nanoparticles. First, the effect of the magnet on the aqueous liquid depends on the proximity of the liquid to the magnet. For large sample volumes, which may particularly be required when the target analyte is of low concentration, the magnet may not be in sufficiently close proximity to the entire sample to ensure that all of the magnetic beads or nanoparticles are removed from the liquid and that the supernatant is free of magnetic beads and nanoparticles.

Second, detection of weak fluorescence or of small differences in luminescence intensity, which may be required to accurately detect or quantify target analytes at low concentrations, is best performed with photomultiplier tubes (sometimes known as PMTs) which have higher sensitivity than other light detectors. PMTs are sensitive to magnetic fields and for good performance should not be operated near magnets. Thus, use of PMTs with magnetic beads or nanoparticles is problematic or impossible.

Third, magnetic nanoparticles may not be compatible with all samples, for example, samples that contain ferromagnetic substances may not be useable with magnetic particles.

Fourth, particles, whether magnetic or not, that are used for two-phase assays with have liquid and solid components can have a tendency to aggregate. Particle aggregation can decrease the accuracy of the assay.

Fifth, samples, especially samples from animals, plants, or the environment, may contain chemical compounds that interfere with detection of the target analyte.

The fourth and fifth problems can be in part due to the issue of quenching of a detector molecule's luminescence. More specifically, typical assays rely on the luminescence (such as fluorescence or phosphorescence) of a detector compound, which, depending on the assay type, can be turned on or turned off when an affinity group binds to a target analyte. Thus, accuracy of luminescence-based assays is compromised if luminescence is quenched by processes other than those related to the binding of a target analyte to the affinity groups. Aggregation of particles (such as those containing affinity groups) can effect self-quenching of detector compounds.

Magnetic beads are in most cases luminescence quenchers, meaning that magnetic beads can lower assay sensitivity even without aggregation. Similarly, some components of samples other than target analytes can quench detector compound luminescence. The result of this quenching, which can include losses of assay accuracy, precision, and sensitivity, limit the use of fluorescence immunoassays to detection of compounds at relatively high concentrations in complex matrices.

Further, with respect to problems involving compounds other than target analytes in the sample, such compounds can also interfere with the assay by mechanisms other than quenching, such as competitively binding to the target analyte and binding (even without quenching) to detector compounds, among other mechanisms of interference.

Still further, when particles that are not magnetic are used, they must be removed from the assay by one or more manipulations, such as centrifuging, filtration, or both. Such manipulations increase the chance of user error.

Notably, many of these problems, such as the problems with aggregation, quenching, and interference of compounds other than the target analyte, are not specific to magnetic particles but can be problems with any type of particle used in a heterogeneous assay. Thus, another problem is to reduce or eliminate sample interference, self-quenching, or both, in an multiplexing assay, that is, a single assay that can detect more than one type of analyte.

Still another problem is to provide a heterogenous assay that permits multiplexing, that is, the detection of more than one analyte in the same assay.

The problems above all relate to a general problem of how to detect a target analyte, and particularly a target analyte that is present in low concentration, requires a high degree of sensitivity, or both. More particularly, the problems relate to issues that decrease the sensitivity, accuracy, or precision of heterogeneous assays, or that make such assays less robust.

One type of target analyte to which these problems pertain is mycotoxins, such as aflatoxin. Other types of targets to which these problems may pertain include whole cells, such as bacterial cells, viruses, virions, toxins, proteins and peptides.

Solutions to one or more of the foregoing problems, and in some cases to other problems, can be found in the present disclosure, which provides a composition and method of detecting an analyte using the composition. An analyte that is being detected is typically referred to as a "target analyte." Particularly, the present composition and method allow for multiplexing, which is simultaneously measuring levels of more than one analyte at once.

The compositions include a first plurality of hollow glass bubbles that are covalently attached to first affinity groups. The plurality of first glass bubbles are embedded in a first polymer matrix material. The first polymer matrix material is a constituent of a film that is disposed on a substrate. Optionally, additional affinity groups, such as second, third, fourth, etc. affinity groups, can also be employed.

In particular embodiments of the herein described composition and method that are configured for multiplexing, it is possible to sequentially bind more than one target analyte in the same assay rather than simultaneously bind all target analytes. This can solve yet another problem, specifically, the problem of interference that occurs between different affinity groups when multiple affinity groups are present in the multiplexing assay at the same time.

### Affinity Groups

Any suitable affinity group can be used as the first affinity group depending on the analyte being detected. When additional affinity groups, such as second, third, fourth etc. affinity groups, are employed, they can be independently selected from the same types of affinity groups described herein.

Typically, the affinity groups are be selected to bind to an analyte, such as a target analyte. Examples include antibodies, antibody substrates, and the like. Examples include antibodies, Alternatively, any of the affinity groups can be selected to bind to quenchers or other materials that may interfere with the detection of a target analyte. Examples include sorbents, zeolites, and chelating agents. It is also possible to select affinity groups to bind to compounds or materials other than target analyte, for example, affinity groups can be selected to bind to compounds or materials that may interfere with the assay in order to reduce or eliminate interference with the assay.

One type of affinity group that can be used is an antibody. Appropriate antibodies can be selected by one of skill in the art depending on the target analyte or target analytes that is being detected. Examples include rabbit anti-mouse IgG antibody, anti-cortisol antibodies, anti-fumonisin antibodies, and anti-aflatoxin B 1 antibodies. Suitable antibodies can be obtained from a variety of sources, including Jackson ImmunoResearch, West Grove, PA, Abeam, Cambridge, MA, Lifespan Biosciences, Seattle, WA, and Creative Diagnostics, Shirley, NY.

Another type of affinity group that can be used is an aptamer, i.e., a short polynucleotide. Examples of aptamers include anti-aflatoxin and anti-ochratoxin aptamers, which are available from Aptagen, Jacobus, PA. The specific aptamer will likewise depend on the target analyte or target analytes that is being detected.

In some cases, one or more target analyte molecules, such as a heavy metal or a small organic molecule, can be used as the affinity group. Herein, a small organic molecule is one having a molecular weight of no greater than 5000 grams/mole.

### Hollow Glass Bubbles

The hollow glass bubbles used herein require a density that will permit them to float in the liquid used in the assay, which is typically an aqueous liquid. Thus, the hollow glass bubbles have a density of less than 0.60 gram/mL, optionally no more than 0.40 gram/mL. While there is no lower limit that is required for the glass bubbles, in practice most glass bubbles that are employed have a density that is no less than 0.05 gram/mL. Thus, particular hollow glass bubbles that are employed have a density in a range of 0.05 to 0.40 gram mL.

The hollow glass bubbles have a span of less than 1.0, less than 0.8, or less than 0.7. In certain embodiments, the hollow glass bubbles have a span of at least 0.1, at least 0.2, at least 0.3, at least 0.4, or at least 0.5. In preferred embodiments, the hollow glass bubbles have a span of 0.1 to 1.0, 0.1 to 0.8, 0.1 to 0.7, or 0.1 to 0.5. Typically, the narrower the span, the higher the homogeneity of the hollow glass bubbles, the shorter the assay, and the better the repeatability of the assay.

The size of the hollow glass bubbles can be selected depending on the needs of the particular assay in which they are being used. Considerations include the speed at which the hollow glass bubbles need to be released from the polymer matrix in which they are embedded, minimizing aggregation, the speed at which the hollow glass bubbles float to the top of the liquid, and in some cases light scattering effects. Most commonly hollow glass bubbles employed herein have an average diameter of at least 30 micrometers, and optionally least 40 micrometers.

Typically, the hollow glass bubbles have an average diameter of no more than 80 micrometers. In particular cases, the hollow glass bubbles have an average diameter that is from 30 to 80 micrometers, optionally from 40 to 80 micrometers. Typically, the larger the hollow glass bubbles, the faster they float to an upper concentrated portion of the liquid for a given density.

Suitable hollow glass bubbles are commercially available from a variety of sources. These include, for example, those available under the designations XLD3000 silica bubbles and 3M^{™} Glass Bubbles S60HS available from 3M Company, St Paul, MN. Typically, commercially available hollow glass bubbles are fractionated to obtain the desired size distribution as determined by average diameter and span. Such fractionated hollow glass bubbles contribute to the repeatability of the assay.

The advantage of using glass bubbles as a solid support compared to other particle-based assays resides in their inherent buoyancy property that allows for an easy, tunable, and rapid separation from solution. Thus, unlike other particles, a partial separation of the glass bubbles from the liquid components of an assay can be effected without applying mechanical forces such as agitation or centrifugation, and without applying magnetic forces. Instead, the glass bubbles can be allowed to float to the surface of the liquid, allowing the lower portion of the liquid without the glass bubbles to be tested for the presence of analyte. While it is not always necessary to do so, it is still possible to easily collect or harvest the glass bubbles that are bound to the target analyte for analysis.

An additional differentiator of the present methods is that the fluorescence signal can be detected from assay components that are in the liquid phase, and not from the components than on a collected solid phase. This eliminates the requirement for washing the solid phase, which is a significant advantage in both time to result and in the elimination of operator error.

The hollow glass bubbles can be covalently attached to the affinity groups by any method for functionalizing glass. Many of such methods are known, and the appropriate method depends on the nature of the affinity group. In some case, the hollow glass bubbles are activated to facilitate affinity group attachment. Activation can be accomplished, for example, by silanization.

Conventional silanization reagents (e.g., 3-glycidoxypropyltrimethoxysilane) and techniques are known in the art. Alternatively, commercially available functionalized glass bubbles, such as glycidyl-functionalized H20 silica bubbles commercially available from 3M Company, St. Paul, MN, can be used.

The affinity group may be further attached, such as complexed with, or noncovalently bonded to, a detector compound that can be detected, e.g., by fluorescence. For convenience, the affinity group is described herein as being distinct from the detector compound/group even when the detector compound or detector group is attached to the affinity group.

### Detector Compound Molecules and Detector Groups

The detector compound molecules include a detector group that can be detected, e.g., by luminescence and particularly by fluorescence, and a complementary group. Such detector compounds or groups are typically detectable at a specific wavelength, such as the wavelength of maximum emission (λₘₐₓ) of the detector group. When multiple detector groups or detector compounds are employed, such as in a multiplexing composition or method, a first detector groups or compounds can be detected at a first wavelength, a second detector groups or compounds can be detected at a second wavelength, a third detector groups or compounds can be detected at a third wavelength and so forth.

A variety of detector compounds and detector groups are known for use with biological assays. In principle, any detector compound or group can be used with the compositions and methods of this disclosure so long as it is compatible with the assay conditions; incompatibilities can include undergoing chemical reaction with the target analyte or another component of the assay, being unstable under the assay conditions, and so forth. For example, the amine-reactive TEXAS RED-X succinimidyl ester can be used to create bright red-fluorescent detector compounds with excitation/emission maxima of approximately 595/615 nanometers (nm).

Fluorescein isothiocyanate can be used to provide fluorescence at 520 nm emission, with 488 nm excitation. Many other examples will be known to the skilled artisan who can select the appropriate detector group and compound depending on the requirements of the assay, the detection system to be used, and other factors.

In any embodiment herein that involves detection or detectability at a first wavelength, the first wavelength is particularly a wavelength in the visible or UV portion of the electromagnetic spectrum, more particularly in the visible portion of the electromagnetic spectrum, and most particularly 600 nm to 850 nm.

In any embodiment herein that involves detection or detectability at a second wavelength, the second wavelength is particularly a wavelength in the visible or UV portion of the electromagnetic spectrum, more particularly in the visible portion of the electromagnetic spectrum, and most particularly 600 nm to 850 nm.

In any embodiment herein that involves detection or detectability at a third wavelength, the third wavelength is particularly a wavelength in the visible or UV portion of the electromagnetic spectrum, more particularly in the visible portion of the electromagnetic spectrum, and most particularly 600 nm to 850 nm.

Detection of detector compounds or groups can be accomplished using luminometers, and particularly fluorimeters. A variety of these are known; they typically feature photomultiplier tubes or charged coupled devices (CCD) for detecting luminescence, such as fluorescence.

### Substrates, Films, and Polymer Matrices

The composition includes a film that itself comprises a first polymer matrix in which a first plurality of hollow glass bubbles is embedded. The film is on a substrate, which is typically a vessel, such as a PCR tube, cuvette, Eppendorf tube, test tube, or the like, in which an assay is to be conducted.

The embedded hollow glass bubbles need not be in any particular disposition within the polymer matrix, although certain dispositions might provide more desirable results depending on the assay in which the composition will be used. For example, it is possible for the hollow glass bubbles to be located mostly on the surface of the polymer matrix, to be distributed homogeneously or nearly homogeneously throughout the polymer matrix, or to be distributed in some other way in the polymer matrix. For example, if required, the hollow glass bubbles can be distributed so as to provide first-order release from the polymer matrix,

With regard to polymer matrix materials, the terms "over" and "under" are used to refer to refer to the proximity of a particular matrix material or layer of matrix material to the substrate on which the film is disposed, but do not necessarily indicate adjacency. For example, if a first polymer matrix is disposed "over" a second polymer matrix then the second polymer matrix is closer to the substrate than the first polymer matrix; however, the first and second polymer matrices are not necessarily adjacent to one another nor is the second polymer matrix necessarily adjacent to the substrate.

The first polymer matrix material is designed to release the first plurality of glass bubbles from the matrix, for example into a liquid composition. Thus, the film and first polymer matrix materials are usually disposed on a surface of a vessel, such as a cuvette, Eppendorf tube, PCR tube, or the like, into which the glass bubbles can be released. The film and first polymer matrix materials can be disposed on the surface by using a variety of techniques known in the art, such as solvent casting and subsequently drying a solution or suspension of the film and first polymer matrix materials and the glass bubbles , by admixing the glass bubbles with a pre-cured adhesive, placing the admixture on the surface, and allowing it to cure, in which case the cured adhesive becomes the polymer material and film, and the like.

A second polymer matrix material can also be employed, for example, it can be disposed on at least a part of the first polymer matrix. Third, fourth, and further polymer matrix materials can also be employed, each disposed on at least a part of an underlying polymer matrix material. Anu of the various polymer matrix materials can have glass bubbles disposed with them, or they can have no glass bubbles but rather be employed to control the release of glass bubbles from an underlying polymer matrix material.

For example, any of the polymer matrix materials can be selected from immediate release polymers, extended release polymers, and enteric polymers. Immediate release polymer matrixes release all of the glass bubbles from the polymer matrix very quickly, such as in no less than five minutes, no less than two minutes, no less than one minute, or no less than thirty seconds, from the time that a liquid, such as an aqueous liquid, contacts the film, and can include one or more of hydroxypropyl methylcellulose, hydroxypropyl cellulose, hydroxyethyl cellulose, methylcellulose, sodium carboxymethyl cellulose, polyvinyl pyrrolidone, polyvinyl pyrrolidone-polyvinyl acetate copolymer, polyvinyl alcohol, copolymer of polyvinyl alcohol and polyvinyl acetate, copolymer of polyvinyl alcohol and polyethylene glycol, water-soluble (meth)acrylic polymers, water-soluble (meth)acrylic copolymers, water-soluble copolymers of (meth)acrylates and polyethylene glycol, and polyethylene glycol.

Extended release polymer matrixes release the glass bubbles over an extended period of time, such as over at least two minutes, at least five minutes, at least ten minutes, at least twenty minutes, or longer, and can include one or more of poly(gly colic acid), poly(lactic acid), poly(lactic-co-glycolic acid), poly(capralactone), poly(ortho ester), poly(anhydride), poly(amide), poly(ester amide), poly(posphoester), chitosan, hyaluronic acid, poly(alkyl cyanoacrylate), enteric polymer, or enzyme-degradable polymer. In some cases, the matrixes can be chemically modified or have some degree of cross-linking in order to control the release rate.

Delayed release polymer matrixes can afford a delay between the time a liquid contact the film and the time that the release of the glass bubble. For example, a delayed release matrix might include an immediate release layer with an overlayer having no glass bubbles such that no glass bubbles are released until the overlayer is dissolved or disintegrated.

Typically, the various polymer matrix materials are water soluble or water degradable, but it is also possible to have matrix materials that are enzyme degradable, that degrade or dissolve only at particular pHs, temperatures, or salt concentrations, or that are neither soluble nor degradable but that release glass beads in other ways, such polymer matrix materials that have pores through which the glass beads can be released.

In cases where there is a need to remove compounds or materials from the sample matrix, for example when such compounds or materials may interfere with the assay, a multilayer film can be employed. In one example, a first polymer matrix, such as a controlled or extended release polymer matrix, can be disposed under a second polymer matrix. The first polymer matrix can be an extended or controlled release matrix and can have a plurality of glass bubbles with covalently attached affinity groups selected to bind to the target analyte embedded within it. The second polymer matrix, which can be an immediate release polymer, can have embedded within it a plurality of hollow glass bubbles with covalently attached affinity groups selected to bind to the interfering compounds or materials. In use, after a liquid, typically an aqueous liquid, containing the sample contacts the film, the second polymer matrix will release its hollow glass bubbles first. Those hollow glass bubbles will float to the surface while binding to the interfering compounds or materials, thus removing the interfering compounds or materials from the liquid portion of the sample. Next, the first polymer matrix will release the hollow glass bubbles with affinity groups that bind the target analyte. In this way, interfering compounds or materials can be removed before the part of the assay that involves binding the target analyte to affinity groups commences.

In some embodiments where the composition is adapted for multiplexing, the hollow glass bubbles are multiplexed. In such cases, there can be more than one affinity group covalently bound to the hollow glass bubbles. This can be implemented in at least two ways. First, the plurality of hollow glass bubbles can have multiple affinity groups, such as a first affinity group and a second affinity group (and optionally a third, fourth, fifth, sixth, seventh, etc.) bound the glass bubbles so that the same glass bubble may have more than one type of affinity group bound to it.

Another way to effect multiplexing can use at least two pluralities of glass bubbles. The first plurality has a first affinity group covalently bound to it, and the second plurality has a second affinity group, which is different from the first affinity group, covalently bound to it. It is also possible to use third, fourth, fifth, sixth, seventh, etc. affinity groups, each covalently attached to a distinct plurality of glass beads. A combination of the two approaches is also possible, for example, a first plurality of glass bubbles can be covalently attached to a first affinity group and a second affinity group, and a second plurality of glass bubbles can be attached to a third affinity group and a fourth affinity group (further pluralities of glass bubbles and further affinity groups can also be used).

When two pluralities of hollow glass bubbles are employed, they can be in the same or different polymer matrices. For example, a first plurality of hollow glass bubbles can be in a first polymer matrix and a second plurality of hollow glass bubbles can be in a second polymer matrix that is over the first polymer matrix. The result is that the second plurality of hollow glass bubbles will be released before the first plurality. This can be advantageous for some multiplexing assays where it can be useful to bind multiple target analytes sequentially in the same assay rather than simultaneously. For example, this configuration and method of detection is particularly useful in cases where the second affinity group has some cross-affinity for the first target analyte because it allows the first target analyte to be bound before release of the second affinity group thus preventing exposure of the first target analyte to the second affinity group.

Combinations of these approaches can also be employed. For example, when two polymer matrices are employed as described above, a third polymer matrix having a third plurality of glass bubbles that are covalently attached to a sorbent can be disposed over the first and second polymer matrixes. In this way, the sorbent can first remove interfering compounds or materials, and then the first and second pluralities of glass bubbles.

In embodiments wherein the multiplexed hollow glass bubbles include two different pluralities of covalently attached affinity groups, the composition includes: a plurality of first detector compound molecules not covalently bonded to the plurality of multiplexed hollow glass bubbles, the first detector compound molecules including a first detectable group that is detected at a first wavelength; and a plurality of second detector compound molecules not covalently bonded to the plurality of multiplexed hollow glass bubbles, the second detector compound molecules including a second detectable group that is detected at a second wavelength that is different than the first wavelength.

In embodiments wherein the multiplexed hollow glass bubbles include three or more different pluralities of covalently attached affinity groups, the composition includes: a plurality of first detector compound molecules not covalently bonded to the plurality of multiplexed hollow glass bubbles, the first detector compound molecules including a first detectable group that is detected at a first wavelength; a plurality of second detector compound molecules not covalently bonded to the plurality of multiplexed hollow glass bubbles, the second detector compound molecules including a second detectable group that is detected at a second wavelength that is different than the first wavelength; and a plurality of third (and optionally fourth, fifth, sixth, seventh, etc.) detector compound molecules not covalently bonded to the plurality of multiplexed hollow glass bubbles, the third (and optionally fourth, fifth, sixth, seventh, etc.) detector compound molecules including a third (and optionally fourth, fifth, sixth, seventh, etc.) detectable group that is detected at a third (and optionally fourth, fifth, sixth, seventh, etc.) wavelength that is different than the first or second wavelength (or any of the other wavelengths).

### Samples and Target Analytes

The compositions and methods as described herein can be applied to assay for any target analyte that can be bound by an affinity group. Examples include antibodies, substrates, small molecules, and heavy metals, among others. Many target analytes are those that contaminate food (e.g., animal feed) and water (i.e., a food or water contaminant), and those used as biomarkers.

However, in principle any target analyte can be detected so long as at least some of the target analyte in the sample can bind to the affinity group.

Heavy metals are one type target analyte that can be detected. Particular heavy metals are metals or metalloids having an atomic number of greater than 20. Typically, such heavy metal has an atomic number of no greater than 92. Examples include Pb, As, and Hg.

The methods are particularly effective for small organic molecules. Such small organic molecules have a molecular weight of no greater than 5000 grams/mole. Examples of such small organic molecules include: mycotoxins, such as aflatoxins (e.g., Bl, B2, Gl, G2, and Ml), trichothecenes (e.g., deoxynivalenol, HT-2, and T-2), zearalenone, ochratoxins (e.g., A, B, C, and TA), fumonisins (e.g., B 1, B2, B3, and B4), and patulin; shellfish toxins, such as saxitoxin, okadaic acid, domoic acid, and dinophysistoxin derivatives; hormones, such as cortisol, progesterone, estradiol, androstenedione, diethylstilbestrol, ractopamine, clenbuterol, stanozolol, triamcinolone, zilpaterol, zeranol, and trenbolone; antibiotics and antivirals, such as beta-lactams, tetracyclines, sulfonamides, nitrofurans, ciprofloxacin, chloramphenicol, acyclovir, triclosan, streptomycin, amphenicol, fhmixin, florfenicol, fluoroquinolone, gentamycin, lincomycin, neomycin, tylosin, diclazuril, dimetridazole, colistin, and dapsone; biomarkers, such as coprostanol, cholesterol, and creatinine; pesticides, such as atrazine, N,N-diethyl-meta-toluamide (DEET), glyphosate, polychlorinated biphenyls (e.g., DDT and DDE), bromacil, avermectin, organophosphates, and N-methylcarbamates; drugs of abuse, such as opioids (e.g., fentanyl and heroin), cocaine, tetrahydrocannabinol (THC), and amphetamines; and other bioactive compounds, such as dexamethasone, albuterol, caffeine, warfarin, ibuprofen, codeine, lidocaine, celecoxib, benzatropine, metoprolol, omeprazole, nadolol, statins (e.g., atorvastatin and simvastatin), lisinopril, ketamine, and nandrolone. Such small organic molecules do not include proteins. Particularly important small organic molecules to detect include mycotoxins.

The present disclosure also provides methods of detecting an analyte, such as a contaminant, in a sample. These methods include: a) providing a composition that includes: a liquid including water; a plurality of first hollow glass bubbles in the liquid, the first hollow glass bubbles having a a plurality of covalently attached first affinity groups that are covalently attached to at least some of the plurality of first hollow glass bubbles; and a plurality of first detector compound molecules not covalently bonded to the plurality of first hollow glass bubbles, the first detector compound molecules including a first detectable group that is detected at a first wavelength; b) adding the sample to the composition, the sample containing first free target analyte molecules; c) allowing the first free target analyte molecules to bind to the first affinity groups or the first detector compound molecules; d) allowing the plurality of first hollow glass bubbles to float to an upper concentrated portion of the liquid; and e) measuring an amount of the first detector compound molecules in a second portion of the liquid that is below the upper concentrated portion, without removing the plurality of first hollow glass bubbles from the liquid.

In this method, the first hollow glass bubbles have: a density of less than 0.60 gram/mL; a span of less than 1.0; and a plurality of covalently attached first affinity groups. It is understood that not all the bubbles may float to the upper concentrated portion; however, greater sensitivity results from more efficient separation.

The methods described herein may be used for detecting heavy metals and small organic compounds. Such metals and compounds are those typically found contaminating food (e.g., animal feed) and water (i.e., a food or water contaminant), and those used as biomarkers.

Herein, a "heavy metal" is a metal or metalloid having an atomic number of greater than 20 (calcium). Typically, such heavy metal has an atomic number of no greater than 92 (uranium). Examples include Pb (lead), As (arsenic), and Hg (mercury).

The methods are particularly effective for small organic molecules. Such small organic molecules have a molecular weight of no greater than 5000 grams/mole. Examples of such small organic molecules include: mycotoxins, such as aflatoxins (e.g., Bl, B2, Gl, G2, and Ml), trichothecenes (e.g., deoxynivalenol, HT-2, and T-2), zearalenone, ochratoxins (e.g., A, B, C, and TA), fumonisins (e.g., B 1, B2, B3, and B4), and patulin; shellfish toxins, such as saxitoxin, okadaic acid, domoic acid, and dinophysistoxin derivatives; hormones, such as cortisol, progesterone, estradiol, androstenedione, diethylstilbestrol, ractopamine, clenbuterol, stanozolol, triamcinolone, zilpaterol, zeranol, and trenbolone; antibiotics and antivirals, such as beta-lactams, tetracyclines, sulfonamides, nitrofurans, ciprofloxacin, chloramphenicol, acyclovir, triclosan, streptomycin, amphenicol, fhmixin, florfenicol, fluoroquinolone, gentamycin, lincomycin, neomycin, tylosin, diclazuril, dimetridazole, colistin, and dapsone; biomarkers, such as coprostanol, cholesterol, and creatinine; pesticides, such as atrazine, N,N-diethyl-meta-toluamide (DEET), glyphosate, polychlorinated biphenyls (e.g., DDT and DDE), bromacil, avermectin, organophosphates, and N-methylcarbamates; drugs of abuse, such as opioids (e.g., fentanyl and heroin), cocaine, tetrahydrocannabinol (THC), and amphetamines; and other bioactive compounds, such as dexamethasone, albuterol, caffeine, warfarin, ibuprofen, codeine, lidocaine, celecoxib, benzatropine, metoprolol, omeprazole, nadolol, statins (e.g., atorvastatin and simvastatin), lisinopril, ketamine, and nandrolone. Such small organic molecules do not include proteins. Particularly important small organic molecules to detect include mycotoxins.

The samples that include such analytes can be a solid or a liquid, but are in the form of a liquid when fluorescence intensity is measured. The samples are typically food (e.g., grains such as oats, com, wheat) or water samples. If a solid sample is to be analyzed, the target analyte is preferably extracted using aqueous extraction techniques to avoid handling and disposal of organic solvents. Typical aqueous extraction techniques use surfactant-based extraction. For example, TWEEN 20 and TRITON-X nonionic detergents can be used to extract the target analyte. If interference is observed, this may be overcome by dilution in a buffer (e.g., phosphate buffered saline).

### Bound Antibody Compositions/Methods

The films as described herein and containing one or more polymer matrices and at least a first plurality of glass bubbles can be used in a variety of types of assays. Regardless of the type of assay, a liquid that will release at least some of the hollow glass bubbles from a polymer matrix, e.g., by dissolving the matrix, degrading the matrix, or causing the hollow glass bubbles to elute from the matrix, is contacted with the film.

The liquid can be a sample-containing composition, such as from a sample that is extracted, diluted, or both, as discussed herein. The liquid can also contain free detector compounds, which are detector compounds or groups that are not bound to hollow glass bubbles. The assay methods include contacting a sample, usually as a liquid and most typically a solution, with the film comprising the polymer matrix and the hollow glass bubbles. In some cases the sample can be added as a solid that dissolves in a liquid that releases the hollow glass bubbles from the polymer matrix.

Most assays also employ a plurality of at least first detector compound molecules not covalently bonded to the plurality of first hollow glass bubbles. Some assays, such as multiplexing assays, may also deploy a plurality of second, third, fourth, or further detector compound molecules not covalently bonded to the plurality of first hollow glass bubbles. Any of the detector compound molecules not covalently bonded to the plurality of first hollow glass bubbles are typically contacted with the polymer matrix in liquid form, such as by adding them as a solution or dispersion to a vessel on which the film containing the polymer matrix is disposed. In this case, they can be added in the same liquid as the sample or in a different liquid. It is also possible to dispose the detector compound molecules not covalently bonded to the plurality of first hollow glass bubbles within the polymer matrix to be released along with the glass bubbles.

The bound antibody methods of the present disclosure are typically competitive binding (e.g., competitive complexation) assays, wherein detector compounds compete with target analytes present in a liquid sample to complex with a limited number of affinity group, typically antibody, binding sites available on the hollow glass bubbles. After binding, the hollow glass bubbles float to an upper concentrated portion of the liquid, and the luminescence, typically fluorescence, emitting from the unbound (i.e., free) detector compound molecules in the liquid below that of the upper concentrated portion is measured. It is understood that not all the glass bubbles may float to the upper concentrated portion of the liquid.

The compositions described herein can be used in either elution or depletion assay. In the elution method, the hollow glass bubbles having affinity groups, usually antibodies, bound thereto are complexed with free detector compound molecules before being exposed to the sample. This can be accomplished, for example, by initially contacting a liquid containing the free detector compound molecules with the polymer film to release hollow glass bubbles and later adding the sample containing the target analyte. The target analyte in the sample will displace, or elute, the detector compound molecules from the bubbles. In the depletion method, the detector compound molecules are added to a liquid sample containing the target analyte and then, after release of the glass bubbles from the polymer matrix, the two compete for binding to the affinity group, typically antibody, on the glass bubbles.

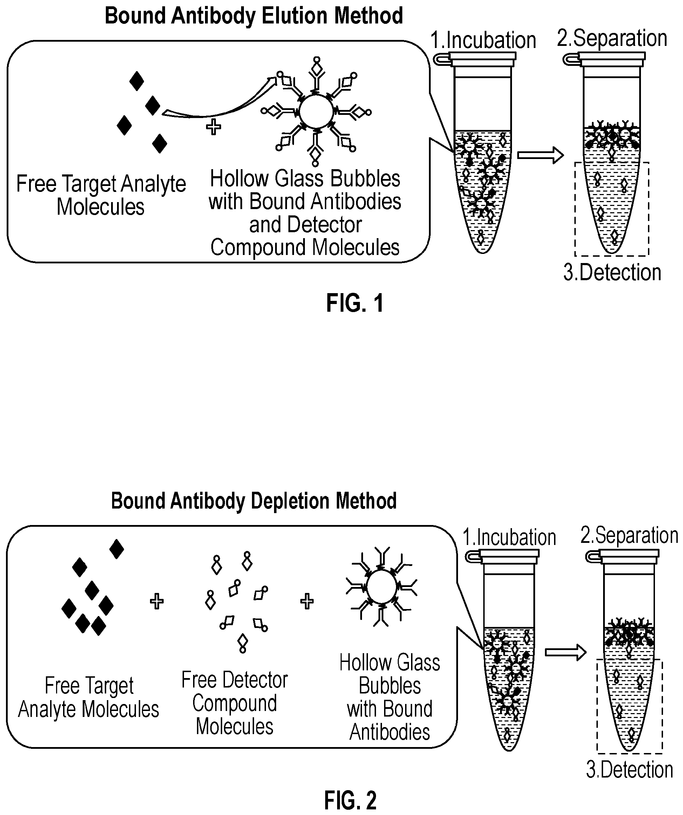

In the elution method, as shown in FIG. 1, antibodies are the affinity groups that are covalently attached to the hollow glass bubbles. The antibodies are complexed with the detector compound molecules prior to contact with free target analyte molecules, thereby forming one type of bound antibody composition. The detector compound molecules and the target analyte molecules both include a complementary group allowing them to compete for binding to the bound antibodies. In this method of detecting an analyte, the method includes: providing a bound antibody composition (that includes hollow glass bubbles with first bound antibodies as the affinity groups, which are complexed with first detector compound molecules); adding a sample to the composition, the sample containing first free target analyte molecules; allowing the first free target analyte molecules to bind to the first bound antibodies (thereby displacing or eluting the first detector compound molecules from the first bound antibodies covalently bonded to the hollow glass bubbles); allowing the plurality of first hollow glass bubbles to float to an upper concentrated portion of the liquid; and measuring an amount of the (displaced) first detector compound molecules in a second portion of the liquid that is below the upper concentrated portion, without removing the plurality of first hollow glass bubbles from the liquid. In Figure 1, a first detector compound can be detectable (i.e., a detector group bonded to a complementary group) initially are complexed with bound antibodies.

In the depletion method, as shown in FIG. 2, antibodies are the affinity groups that are covalently attached to the hollow glass bubbles. A competition is set up between target analyte molecules and detector compound molecules for binding to bound antibodies. In the composition for use in this method, the first detector compound molecules include a first complementary group and the first free target analyte molecules include the first complementary group, wherein the first detector compound molecules compete with the first free target analyte molecules for complexation with the first bound antibodies. In this method of detecting an analyte, the method includes: providing a bound antibody composition and free detector compound molecules; adding
a sample to the composition, the sample containing first free target analyte molecules; allowing the first free target analyte molecules to bind to the first bound antibodies (thereby competing with the first free detector compound molecules); allowing the plurality of first hollow glass bubbles to float to an upper concentrated portion of the liquid; and measuring an amount of the (remaining free) first detector compound molecules in a second portion of the liquid that is below the upper concentrated portion, without removing the plurality of first hollow glass bubbles from the liquid.

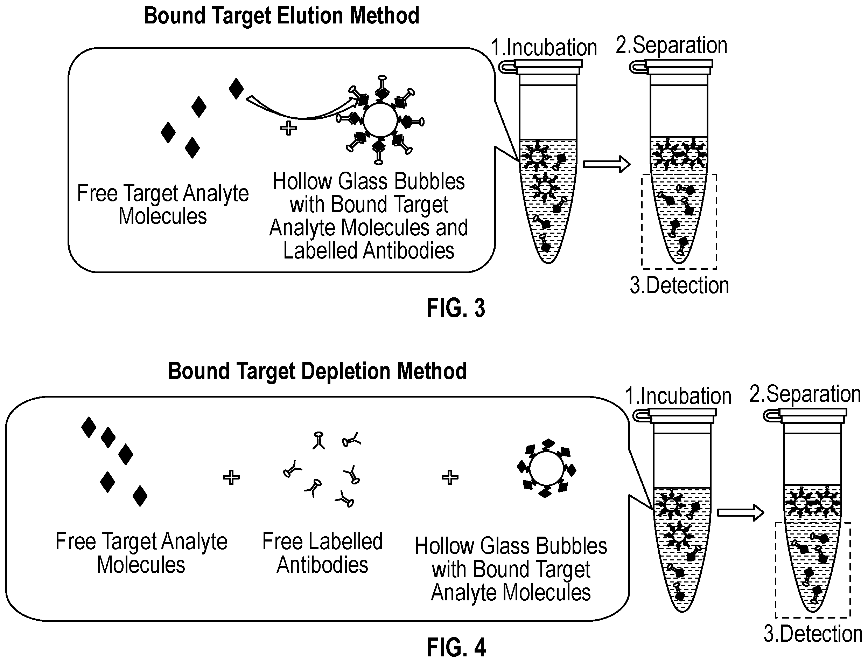

In Figure 2, the detector compound initially is in the form of free molecules. In the Bound Antibody Depletion Method target analyte molecules are covalently bonded to, or otherwise bound to, the hollow glass bubbles and complexed with the labelled antibodies, which are thereby attached (but not covalently) to the glass bubbles. In this method of detecting an analyte, the method includes: providing a bound target composition (that includes hollow glass bubbles with bound target analyte molecules as the affinity groups, which are complexed with labelled antibodies); adding a sample to the composition, the sample containing first free target analyte molecules; allowing the first free target analyte molecules to complex with the first labelled antibodies (thereby displacing or eluting the first labelled antibodies from the first bound target analyte molecules attached to the hollow glass bubbles); allowing the plurality of first hollow glass bubbles to float to an upper concentrated portion of the liquid; and measuring an amount of the first detector compound molecules (the displaced first labelled antibodies complexed with initially first free target analyte molecules) in a second portion of the liquid that is below the upper concentrated portion, without removing the plurality of first hollow glass bubbles from the liquid. The complexing steps can optionally, in some cases, be accompanied by agitation, such as shaking, tumbling, mixing, and the like, to maintain the hollow glass bubbles in a dispersed and unaggregated state in the liquid. In Figure 3, detector compound molecules are labelled antibodies, which are initially complexed with bound target molecules, in the Bound Target Elution Method.

In the depletion method, as shown in FIG. 4, a competition is set up between bound target analyte molecules and free target analyte molecules for complexation with labelled antibodies.

The composition for use in this method includes first free labelled antibodies and hollow glass bubbles having first bound target analyte molecules attached thereto. The first bound target analyte molecules compete with the first free target analyte molecules for complexation with the first free labelled antibodies. In this method of detecting an analyte, the method includes: providing a bound target composition (that includes hollow glass bubbles with first bound target analyte molecules as the affinity groups and free labelled antibodies as the first detector compound molecules); adding a sample to the composition, the sample containing first free target analyte molecules; allowing the first free target analyte molecules to complex with the first labelled antibodies (thereby competing with the first bound target analyte molecules); allowing the plurality of first hollow glass bubbles to float to an upper concentrated portion of the liquid; and measuring an amount of the first labelled antibodies complexed with initially free target analyte molecules in a second portion of the liquid that is below the upper concentrated portion, without removing the plurality of first hollow glass bubbles from the liquid. In Figure 4, the detector compound initially is in the form of free labelled antibodies in the Bound Target Depletion Method.

Typically, during detection, the intensity of the fluorescent signal is directly or inversely proportional to the amount of target analyte in a sample. Thus, in such embodiments, the methods described herein can be used to quantify the amount of target analyte. This can be done by comparing the signal of an unknown sample with that of standards (e.g., typically three or more standard samples) of known target analyte concentrations.

The assays and methods as described herein work in essentially the same way when multiplexing is employed. The main difference is that the hollow glass bubbles will be one or more of the type described herein that are useful for multiplexing, and that more than one free detector compound molecules are employed. After the hollow glass bubbles are allowed to float to the surface, detection takes place by measuring luminescence, such as fluorescence, at multiple wavelengths. The measured luminescence at each wavelength corresponds, such as by direct or indirect proportionality, to the concentration of the target analyte associated with the detector compound whose luminescence is measured.

### EXAMPLES

Objects and advantages of this invention are further illustrated by the following examples, but the particular materials and amounts thereof recited in these examples, as well as other conditions and details, should not be construed as limiting. These examples are merely for illustrative purposes only. Unless otherwise stated, all amounts are in weight percent.

### Materials

Polyvinyl alcohol (PVOH, 4-88 grade) was obtained from Kuraray America, Inc., Houston, TX.

XLD3000 glass (silica) bubbles, S60HS glass (silica) bubbles, and glycidyl-functionalized H20 glass (silica) bubbles were obtained from 3M Company, St Paul, MN.

Sodium sulfate and 0.1N NaOH solution was obtained from Avantor, Radnor, PA.

Ethanol was obtained from Decon Labs, King of Prussia, PA.

3-Glycidoxypropyltrimethoxy silane, dry dimethylsulfoxide (DMSO) and ethanolamine were obtained from Alfa Aesar (Haverhill, MA).

Bovine serum albumin (BSA), TWEEN 20 nonionic detergent, fumonisin B 1, aflatoxin B 1 (AFB 1), and cortisol were obtained from Sigma Aldrich, St. Louis, MO.

N-hydroxysuccinimide (NHS) and Ethyl-3-(dimethylaminopropyl)carbodiimide (EDC) were obtained from Thermo Scientific (Rockford, IL).

Acetic acid, acetone, chloroform, dimethylformamide, methanol, and triethylamine were obtained from EMD Millipore, Billerica, MA.

TEXAS RED-X succinimidyl ester, EZ-LINK hydrazide-PEG4-biotin, and QDOT streptavidin sampler kit (Q10151MP) were obtained from Thermo Fisher Scientific, Waltham, MA.

Rabbit anti-mouse IgG, Cy3 labelled, was obtained from Jackson ImmunoResearch, West Grove, PA.

Anti-cortisol antibodies (XM-210) were obtained from Abeam, Cambridge, MA.

Anti-fumonisin antibodies (PY715173) and anti-aflatoxin B 1 antibodies (DMAB2948) were obtained from Creative Diagnostics, Shirley, NY.

Deionized (DI) water was purified using a MILLI-Q water purification system (EMD Millipore, Burlington, MA).

SUPELCLEAN LC-Si SPE cartridges and AMICON Ultra 0.5 mL centrifugal filter units (3000 NMWL) were obtained from Millipore Sigma, St. Louis, MO.

NANOSEP tubes were obtained from Pall Corporation, Port Washington, NY.

Size distribution was measured using a Flex dynamic light scattering system from Microtrac Inc. (Montgomery, PA).

LCMS (liquid chromatography -mass spectrometry) data was recorded on an Agilent 1260 Infinity HPLC system with a 6130 quadrupole LC/MS (Agilent Technologies, Santa Clara, CA).

Fluorescence was measured on either a Tecan Infinite 200 plate reader (Tecan Group, Ltd., Switzerland) or a Synergy Neo2 plate reader (BioTek Instruments, Winooski, VT).

FITC-cortisol (fluorescein isothiocyanate labelled cortisol) was synthesized according to the procedure in M. Pourfarzaneh et al., Clin. Chem. 26(6), 730-733 (1980).

Anti-AFB 1 antibodies were labelled with ALEXA FLUOR 680 according to the instructions provided in the ALEXA FLUOR 680 labelling kit (#A20188) from Thermo Fisher Scientific, Waltham, MA.

### Preparative Example 1 : Glass Bubble Fractionation

1A - Fractionation of XLD3000 glass bubbles: Glass bubbles XLD3000 (3M ID 98-0212-3469-9; 10 grams (g)) were added to 400 milliliters (mL) of deionized (DI) water in a separation funnel. The suspension was shaken and the glass bubbles were allowed to float for 1 minute (min), then 390 mL of liquid (including debris and small glass bubbles) was drained from the bottom.

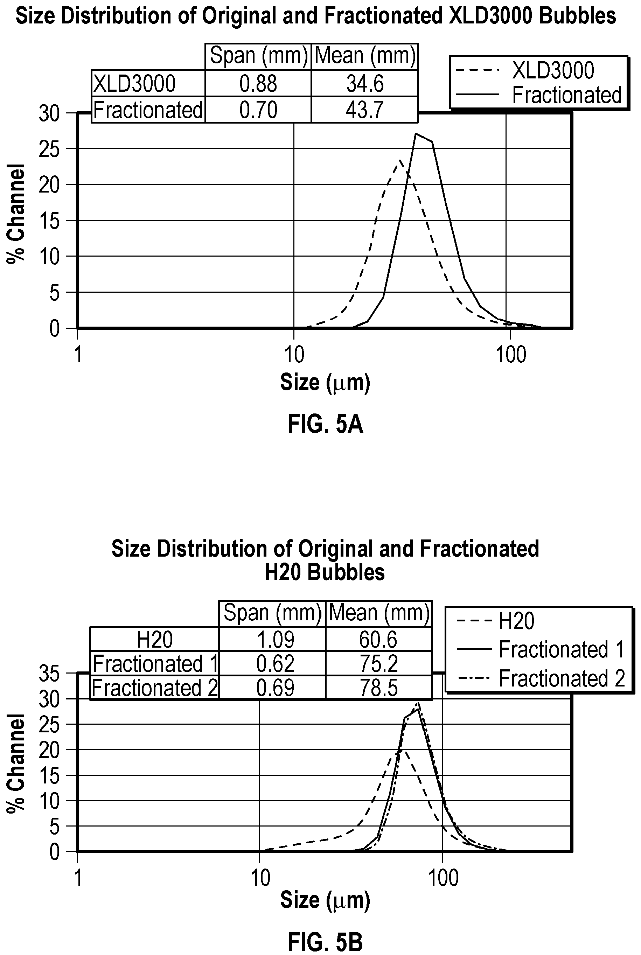

This process was repeated 5 times, after which fractionated glass bubbles were collected by filtering through a Whatman 4 filter under low pressure. The collected glass bubbles were then rinsed with acetone (100 mL) and left to dry at room temperature overnight. The size distribution (as determined by average (i.e., mean) diameter and span) of the fractionated sample is shown in Figure 5A.

IB - Fractionation of H20 glass bubbles: Glass bubbles H20 (3M ID 70-0704-8399-8; 10 grams (g)) were added to 400 milliliters (mL) of DI water in a separation funnel. The suspension was shaken and the glass bubbles were allowed to float for 1 minute (min), then 390 mL of liquid (including debris and small glass bubbles) was drained from the bottom. This process was repeated 5 times, after which fractionated glass bubbles were collected by filtering through a Whatman 4 filter under low pressure. They were then rinsed with acetone (100 mL) and left to dry at room temperature overnight. The size distribution (as determined by average (i.e., mean) diameter and span) of the fractionated sample is shown in Figure 5B.

### Preparative Example 2: Activation and Silanization of XLD3000 Glass Bubbles

Fractionated glass bubbles of Preparative Example 1 (6.4 g; mean = 43.7 micrometers; span = 0.70) were cleaned by gently rotating in 400 mL of 0.1 Normal (N) NaOH (ThermoFisher rotator) at 10 revolutions per minute (rpm) for 3 hours, followed by vacuum filtering with a Whatman #54 filter paper. After rinsing with Milli-Q water, the cleaned glass bubbles were transferred to a plastic dish and dried at room temperature in air overnight.

The cleaned fractionated glass bubbles (500 milligrams (mg)) were added to a 40 mL solution of 95% cthanol/5% 50 millimolar (mM) acetate buffer (pH 5.2) and 300 microliters (pL) of 3 -glycidoxypropyltrimethoxy silane, dispersed by vortexing, and then rotated vertically with a rotator at 20 rpm for a period of time between 30 min and 2 hours. The silanized bubbles were then separated from the solution by vacuum filtration (Whatman #54 filter paper), rinsed with ethanol, transferred to a glass vial, and dried in air at room temperature overnight.

### Preparative Example 3 : Antibody Functionalization of XLD3000 Glass Bubbles

Silanized glass bubbles of Preparative Example 2 (3 mg) were added to a 0.5-mL centrifuge tube and suspended in a solution of 90 microliters of coupling buffer (phosphate buffer with 0.9 Molar (M) sodium sulfate, pH 7.5) and 10 microliters of 1.5 mg/mL IgG reconstituted stock solution (rabbit anti-mouse IgG antibody, Cyanine3 (Cy3) fluorescent dye labelled). The tube was rotated vertically at 20 rpm for 30 min in the dark, after which the glass bubbles were vacuum filtered (Whatman #1 filter paper) and rinsed with 1M NaCl solution followed by deionized (DI) water.

The purpose of this example was to demonstrate efficient binding of antibodies to glass bubbles. Fluorescence microscopy demonstrated consistent coverage of the glass bubbles with the antibodies.

### Preparative Example 4: General Procedure for Antibody Immobilization on H20 Glass Bubbles Pre-coated with Glycidyl Groups

NANOSEP tubes (0.45 micron) were charged with 10 mg of glycidyl-functionalized H20 glass bubbles. A300 microliter volume of antibody solution (approximately 0.1 mg/mL) in coupling buffer (phosphate buffer with 0.9 M sodium sulfate, pH 7.5) was added and the suspension gently rotated at 25 rpm for one hour. Liquid was removed by centrifugation and 0.5 mL of quenching buffer (3 M ethanolamine, pH 9) with 1% BSA was added. The contents of the tube were mixed by rotation at 25 rpm for 1 hour. Liquid was removed by centrifugation and the glass bubbles were washed three times with 0.5 mL of dilution buffer (phosphate buffer, pH 7.4 with 0.1% TWEEN 20 nonionic detergent).

### Preparative Example 5: Synthesis of TEXAS RED-X Fumonisin Conjugate (a Detector Compound)

A 50 microliter sample of 2 mg/mL fumonisin B 1 toxin solution in DMF was mixed with 50 microliters of 2 mg/mL TEXAS RED-X succinimidyl ester in methanol (Invitrogen) and 10 microliters of triethylamine in a small plastic reaction vial. The reaction mixture was allowed to react overnight at room temperature (approximately 20 hours), protected from light. Samples of 10 microliter aliquots of the reaction mixture were loaded onto a SUPELCLEAN LC-Si SPE cartridge and eluted with chloroform/methanol/acetic acid (20:5:0.5). Fractions were collected every 0.5 mL into clean vials and analyzed by LC/MS. Fractions were dried with a gentle stream of nitrogen.

### Preparative Example 6: Immobilization of Aflatoxin B 1 (Target Analyte) on H20 Glass Bubbles

NANOSEP tubes (0.45 micron) were charged with 10 mg of glycidyl-functionalized H20 glass bubbles. A 400 microliter volume of BSA-AFB1 solution (approximately 0.5 mg/mL) in coupling buffer (phosphate buffer with 0.9 M sodium sulfate, pH 7.5) was added and the suspension mixed by rotating at 4 °C overnight. The liquid was then removed by centrifugation and 0.5 mL of quenching buffer (3 M ethanolamine, pH 9) was added and mixed by rotation at 25 rpm for 4 hours. The liquid was removed by centrifugation and the glass bubbles were washed three times with 0.5 mL of dilution buffer (phosphate buffer, pH 7.4 with 0.1% TWEEN 20 nonionic detergent). The glass bubbles were finally resuspended in dilution buffer and stored at 4 °C until use.

### Proof-of-Concept Example 1 : Detection of Aflatoxin by the Bound Target Elution Method

A tube containing 10 mg of aflatoxin B1 -coated glass bubbles in dilution buffer (prepared according to Preparative Example 6) was centrifuged to remove dilution buffer, washed with dilution buffer (phosphate buffer, pH 7.4 with 0.01% TWEEN 20 nonionic detergent), and centrifuged again. A solution of ALEXA FLUOR 680-labelled anti-AFB 1 antibodies (400 microliters of a 10 microgram/mL solution in phosphate buffered saline (PBS)) was added and incubated with rotation for 1.5 hours, protected from light. The glass bubbles were then washed three times with dilution buffer (phosphate buffer, pH 7.4 with 0.01% TWEEN 20 nonionic detergent) to remove excess antibody, resuspended in dilution buffer (300 microliters), and transferred to a 2-mL vial.

The glass bubbles with bound target analyte molecules and complexed labelled antibodies were kept in suspension by vortexing, and 25 microliter quantities were carefully aliquoted into separate clean 1.5-mL tubes. Standard solutions of aflatoxin B1 in dilution buffer (50 ppb, 5 ppb, 0.5 ppb, 0 ppb, 500 microliters of each; ppb = parts per billion) were added to the tubes - one standard solution per tube. Each tube was rotated for 30 min, protected from light. The glass bubbles were allowed to float to the surface while tilting the tube and 200 microliters of liquid from each tube was carefully transferred to a NANO SEP tube and centrifuged to filter out residual glass bubbles before being transferred to one well of a black, half area, 96-well plate.

Fluorescence was measured at 715 nm emission, with 670 nm excitation.

Table 1 shows the average fluorescence signal intensity measured from several replicates of solutions containing the indicated amount of aflatoxin B 1 after 30 min incubation with functionalized glass bubbles. The antibodies tested gave good results with the signal from solution increasing at higher concentration of target in sample, as well as clear discrimination between 0.5 ppb and zero ppb.

**TABLE 1**

| Aflatoxin B 1 Concentration (ppb) | Proof-of-Concept Example 1 (Fluorescence Signal Intensity) |
|---|---|
| 50 | 19,979 |
| 0.50 | 13,285 |
| 0 | 11,870 |

### Proof-of-Concept Example 2: Multiplexed Detection of Fumonisin and Cortisol in the Same Sample Using the Bound Antibody Elution Method

2A - Cortisol bubble preparation: XLD3000 glass bubbles were first silanized with 3-glycidoxypropyltrimethoxysilane as in Preparative Example 2. A NANOSEP tube was then charged with 10 mg of epoxy -functionalized XLD3000 glass bubbles. A solution of anti-cortisol antibodies (XM-210) was prepared by mixing 25 microliters of 2 mg/mL antibody solution and 375 microliters coupling buffer (phosphate buffer with 0.9 M sodium sulfate, pH 7.5), and was then added to the tube containing the glass bubbles. The tube was gently rotated at 25 rpm for 1 hour. The liquid was removed by briefly centrifuging. An aliquot (0.5 mL) of quenching buffer (3 M ethanolamine, pH 9) with 1% BSA was added and the suspension rotated at 25 rpm for 1 hour. The liquid was removed again by brief centrifugation and the glass bubbles were washed three times with 0.5 mL of dilution buffer (phosphate buffer, pH 7.4 with 0.1% TWEEN 20 nonionic detergent). A solution of FITC-cortisol conjugate (500 microliters, 100 nM) was added and the suspension was rotated for 1.5 hours, protected from light. The liquid was removed by centrifugation. The glass bubbles were washed three times with dilution buffer to remove excess conjugate, and then resuspended in 0.25 mL of dilution buffer.

2B - Fumonisin bubble preparation: XLD3000 glass bubbles were first silanized with 3-glycidoxypropyltrimethoxysilane as in Preparative Example 2. A NANOSEP tube was then charged with 10 mg of epoxy -functionalized XLD3000 glass bubbles. A solution of anti-fumonisin antibodies (Creative Diagnostics PY715173) was prepared by mixing 10 microliters of 5.3 mg/mL antibody solution and 390 microliters coupling buffer (phosphate buffer with 0.9 M sodium sulfate, pH 7.5), and was then added to the tube containing the glass bubbles. The tube was gently rotated at 25 rpm for 1 horn. The liquid was removed by briefly centrifuging. An aliquot (0.5 mL) of quenching buffer (3 M ethanolamine, pH 9) with 1% BSA was added and the suspension rotated at 25 rpm for 1 hour. The liquid was removed again by brief centrifugation, and the glass bubbles were washed three times with 0.5 mL of dilution buffer (phosphate buffer, pH 7.4 with 0.1% TWEEN 20 nonionic detergent). A solution of TEXAS RED-X fumonisin conjugate (500 microliters, 100 nM, prepared according to Preparative Example 5) was added and the suspension was rotated for 1.5 hours, protected from light. The liquid was removed by centrifugation. The glass bubbles were washed three times with dilution buffer to remove excess conjugate, and then resuspended in 0.25 mL of dilution buffer.

2C - Multiplexed competitive assay by the elution method: Glass bubble suspensions from 2A and 2B were kept in suspension by vortexing and 25 microliter quantities were carefully aliquoted into clean 1.5-mL tubes. Aliquots of 9 different target sample solutions plus a control solution (200 microliters each of cortisol alone: at 10 ppb, 5 ppb, lppb; fumonisin alone: at 10 ppb, 5 ppb, 1 ppb; cortisol + fumonisin mixture: at 10 ppb of each component, 5 ppb of each component, 1 ppb of each component; and control: at 0 ppb; n = 2 for each sample) were prepared with dilution buffer and individually added to separate tubes containing glass bubbles (a single aliquot added per tube). Each tube was rotated for 1 hour, protected from light. The glass bubbles were allowed to float to the surface while tilting the tube and 180 micro liters of liquid from each tube was carefully transferred to one well of a black, half area, 96-well plate. Fluorescence intensity was measured under two sets of conditions: excitation 488 nm/emission 520 nm (for cortisol detection) and excitation 590 nm/emission 620 nm (for fumonisin detection).

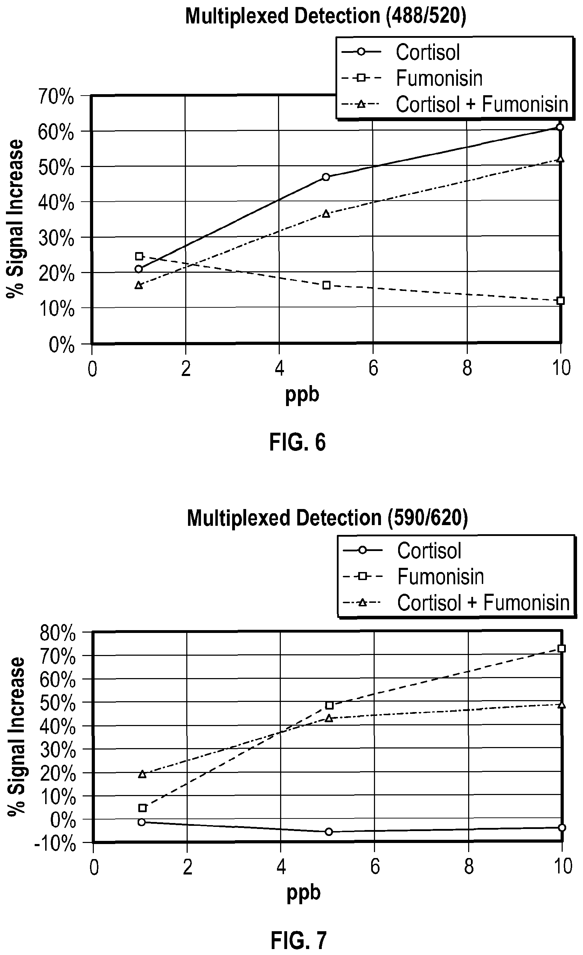

The results are presented in Tables 2 and 3, and Figures 6 and 7, as the percentage increase in signal over the control. The data in Table 2 and Figure 6 clearly demonstrates that a solution containing both cortisol and fumonisin closely tracks the response of a solution containing only cortisol, when interrogated at the appropriate wavelengths for the FITC-cortisol conjugate. The data in Table 3 and Figure 7 clearly demonstrates that a solution containing both cortisol and fumonisin closely tracks the response of a solution containing only fumonisin, when interrogated at the appropriate wavelengths for the TEXAS RED-X conjugate.

**TABLE 2**

| | % Increase in Signal (excitation 488 nm/emission 520 nm) | | |
|---|---|---|---|
| Concentration | Cortisol Only | Fumonisin Only | Cortisol + Fumonisin |
| 10 ppb cortisol, 10 ppb fumonisin, or mixture containing 10 ppb of each | 60.5% | 12.0% | 51.9% |
| 5 ppb cortisol, 5 ppb fumonisin, or mixture containing 5 ppb of each | 46,9% | 16.3% | 36.6% |
| 1 ppb cortisol, 1 ppb fumonisin, or mixture containing 1 ppb of each | 21.1% | 24.3% | 16.5% |

**TABLE 3**

| | % Increase in Signal (excitation 590 nm/emission 620 nm) | | |
|---|---|---|---|
| Concentration | Cortisol Only | Fumonisin Only | Cortisol + Fumonisin |
| 10 ppb cortisol, 10 ppb fumonisin, or mixture containing 10 ppb of each | -4.1% | 72.8% | 48.6% |
| 5 ppb cortisol, 5 ppb fumonisin, or mixture containing 5 ppb of each | -5.8% | 48.6% | 43.3% |
| 1 ppb cortisol, 1 ppb fumonisin, or mixture containing 1 ppb of each | -1.3% | 5.1% | 19.7% |

### Proof-of-Concept Example 3 : Multiplexed Detection of Aflatoxin, Fumonisin, and Cortisol in the Same Sample Using the Bound Antibody Elution Method

3 A - Cortisol bubble preparation: A NANOSEP tube was charged with 12 mg of epoxy- coated H20 glass bubbles. A solution of anti-cortisol antibodies (XM-210) was prepared by mixing 25 microliters of 2 mg/mL antibody solution and 375 microliters coupling buffer (phosphate buffer with 0.9 M sodium sulfate, pH 7.5), and was then added to the tube containing the glass bubbles. The tube was gently rotated at 25 rpm for 1 hour. The liquid was removed by briefly centrifuging. An aliquot (0.5 mL) of quenching buffer (3 M ethanolamine, pH 9) with 1% BSA was added and the suspension rotated at 25 rpm for 1 hour. The liquid was removed again by brief centrifugation, and the glass bubbles were washed three times with 0.5 mL of dilution buffer (phosphate buffer, pH 7.4 with 0.1% TWEEN 20 nonionic detergent). A solution of FITC-cortisol conjugate (400 microliters, 100 nM) was added and the suspension was rotated for 1.5 hours, protected from light. The liquid was removed by centrifugation. The glass bubbles were washed three times with dilution buffer, and then resuspended in 0.3 mL of dilution buffer.

3B - Fumonisin bubble preparation: A NANOSEP tube was charged with 12 mg of epoxy -coated H20 glass bubbles. A solution of anti-fumonisin antibodies (Creative Diagnostics PY715173) was prepared by mixing 10 microliters of 5.3 mg/mL antibody solution and 390 microliters coupling buffer (phosphate buffer with 0.9 M sodium sulfate, pH 7.5), and was then added to the tube containing the glass bubbles. The tube was gently rotated at 25 rpm for 1 hour. The liquid was removed by briefly centrifuging. An aliquot (0.5 mL) of quenching buffer (3 M ethanolamine, pH 9) with 1% BSA was added and the suspension rotated at 25 rpm for 1 hour.

The liquid was removed again by brief centrifugation, and the glass bubbles were washed three times with 0.5 mL of dilution buffer (phosphate buffer, pH 7.4 with 0.1% TWEEN 20 nonionic detergent). A solution of TEXAS RED-X fumonisin conjugate (400 microliters, 100 nM, prepared according to Preparative Example 5) was added and the suspension was rotated for 1.5 hours, protected from light. The liquid was removed by centrifugation. The glass bubbles were washed three times with dilution buffer, and then resuspended in 0.3 mL of dilution buffer.

3C - Aflatoxin B1 bubble preparation: A NANOSEP tube was charged with 12 mg of epoxy -coated H20 glass bubbles. BSA-AFB 1 solution (0.5 mg/mL in coupling buffer (phosphate buffer with 0.9 M sodium sulfate, pH 7.5)) was added to the tube containing the glass bubbles and the tube was gently rotated at 25 rpm for 1 hour. The liquid was removed by briefly centrifuging. An aliquot (0.5 mL) of quenching buffer (3 M ethanolamine, pH 9, without extra BSA), was added and the suspension rotated at 25 rpm for 1 hour. The liquid was removed again by briefly centrifuging, and the glass bubbles were washed three times with 0.5 mL of dilution buffer (phosphate buffer, pH 7.4 with 0.1% TWEEN 20 nonionic detergent). A solution of ALEXA FLUOR 680-labelled anti-aflatoxin antibody (400 microliters, 10 micrograms/mL) was added and the suspension was rotated for 1.5 hours, protected from light. The liquid was removed by centrifugation. The glass bubbles were washed three times with dilution buffer, and then resuspended in 0.3 mL of dilution buffer.

3D - Multiplexed competitive assay by the elution method: Glass bubble suspensions from 3A, 3B, and 3C were combined, allowed to rise, and 0.54 mL of buffer solution was removed from the bottom to leave a glass bubble concentration of 100 mg/mL. The bubbles were kept in suspension by vortexing and 20 microliter quantities were carefully aliquoted into clean 2-mL tubes. Aliquots of 8 different target sample solutions plus a control solution (300 microliters each of cortisol alone: at 50 ppb, 5 ppb; fumonisin alone: at 50 ppb, 5 ppb; aflatoxin alone: at 50 ppb, 5 ppb; cortisol + fumonisin + aflatoxin B1 mixture: at 50 ppb of each component, 5 ppb of each component; and control: at 0 ppb; n = 2 for each sample) were prepared with dilution buffer and individually added to separate tubes containing glass bubbles (a single aliquot added per tube). Each tube was rotated for 30 min, protected from light. The glass bubbles were allowed to float to the surface while tilting the tube. Liquid (130 microliters) from each duplicate was removed, combined, and filtered through a NANOSEP tube. The combined, filtered sample (170 microliters) was transferred to one well of a black, half area, 96-well plate. Fluorescence intensity was measured for the filtered samples under three sets of conditions: excitation 488 nm emission 520 nm (cortisol); excitation 590 nm emission 620 nm (fumonisin); excitation 670 nm emission 715 nm (aflatoxin Bl).

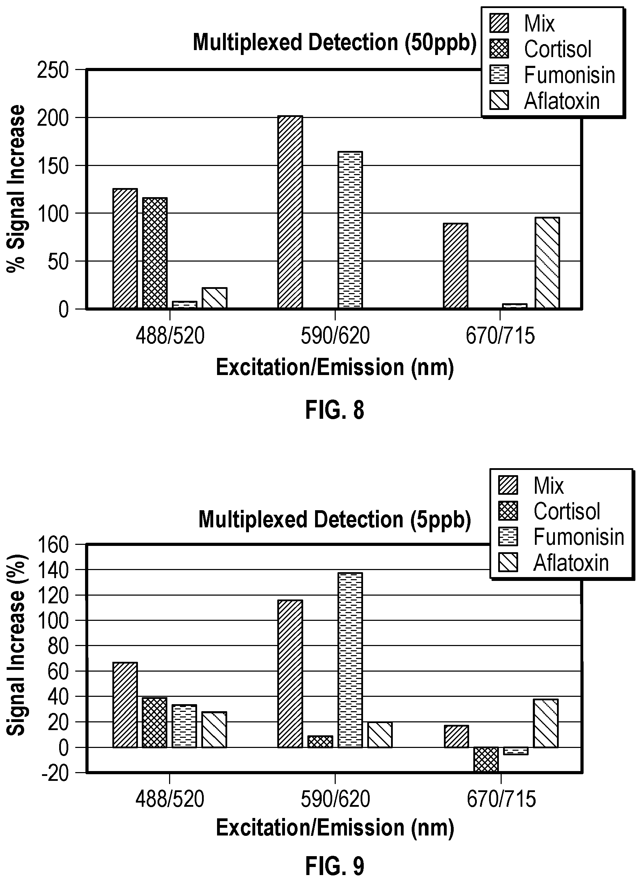

The data is presented in Tables 4 and 5, and Figures 8 and 9, as the percentage increase in signal over the control. Table 4 shows the percent increase in fluorescence at different wavelengths for solutions containing 50 ppb of either cortisol, fumonisin, aflatoxin B 1, or a mixture containing 50 ppb of each component. Table 5 shows the percent increase in fluorescence at different wavelengths for solutions containing 5 ppb of either cortisol, fumonisin, aflatoxin Bl, or a mixture containing 5 ppb of each component. The data clearly demonstrates that all three targets can be easily detected at 50 ppb, and that fumonisin in particular can be easily detected at 5 ppb, even in the presence of other targets and fluorescent conjugates.

**TABLE 4**

| | % Increase in Signal | | | |
|---|---|---|---|---|
| Excitation/emission wavelengths (nm) | Mixture Cortisol+Fumonisin+Aflatoxin | Cortisol Only | Fumonisin Only | Aflatoxin Only |
| 488/520 | 125% | 116% | 8% | 21% |
| 590/620 | 202%, | -9% | 164% | -6% |
| 670/715 | 89% | -18% | 5% | 95% |

**TABLE 5**

| | % Increase in Signal | | | |
|---|---|---|---|---|
| Excitation/emission wavelengths (nm) | Mixture Cortisol+Funionisin+Aflatoxin | Cortisol Only | Fumonisin Only | Aflatoxin Only |
| 488/520 | 66% | 39% | 33% | 27% |
| 590/620 | 116% | 9% | 137% | 20% |
| 670/715 | 17% | -67% | -6% | 37% |

### Proof-of-Concept Example 4: Detection of Fumonisin in Grain Extracts Using Bound Antibody Elution Method

Samples (2 g) of three different grains (oats, com, wheat) were weighed into separate 50- mL centrifuge tubes. Each was treated with 10 mL of a 3% solution of TWEEN 20 nonionic detergent in PBS, shaken by hand for three minutes, and allowed to settle for 30 seconds. The supernatant was decanted, filtered through a 0.45 micrometer syringe filter, and then diluted 10X with PBS. Sufficient quantities of a 10-ppm (parts per million) standard solution of fumonisin in water were added to either grain extract or dilution buffer (phosphate buffer, pH 7.4 with 0.1% TWEEN 20 nonionic detergent) to make individual samples containing 100 ppb, 50 ppb, 5 ppb, and 0 ppb fumonisin.

XLD3000 glass bubbles were first silanized with 3-glycidoxypropyltrimethoxysilane as in Preparative Example 2. NANOSEP tubes were then charged with 5 mg of the epoxy-functionalized XLD3000 glass bubbles each, followed by a solution of anti-fumonisin antibodies (Creative Diagnostics PY715173) prepared by mixing 5 microliters of 5.3 mg/mL antibody solution and 395 microliters coupling buffer (phosphate buffer with 0.9 M sodium sulfate, pH 7.5). The tubes were gently rotated at 25 rpm for 1 hour, after which the liquid was removed by briefly centrifuging and 0.35 mL of quenching buffer (3 M ethanolamine, pH 9) with 1% BSA was added. The resulting suspension was rotated at 25 rpm for one hour. The liquid was removed again by brief centrifugation, and the glass bubbles were washed three times with 0.4 mL of dilution buffer (phosphate buffer, pH 7.4 with 0.1% TWEEN 20 nonionic detergent). A solution of TEXAS RED-X fumonisin conjugate (400 microliters, 100 nM, prepared according to Preparative Example 5) was added and the suspension was rotated for one hour, protected from light. The liquid was removed by centrifugation. The glass bubbles were washed three times with dilution buffer to remove excess conjugate, and then resuspended in 0.2 mL of dilution buffer.

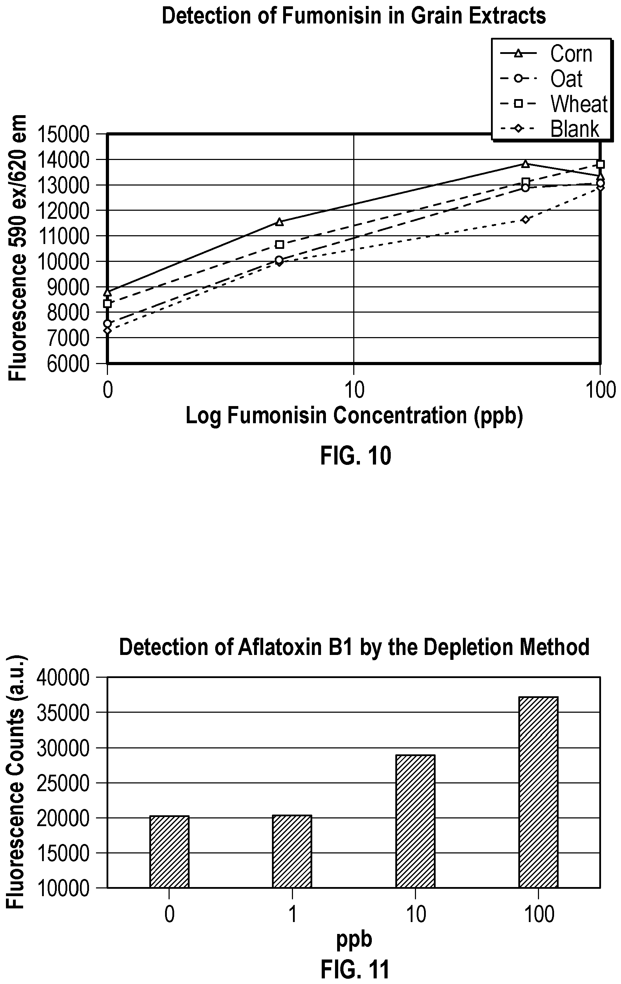

The glass bubbles were kept in suspension by vortexing and 25 microliter quantities were carefully aliquoted into clean 1.5-mL tubes. Target solutions (100 ppb, 50 ppb, 5 ppb and 0 ppb, 250 microliters of each grain extract or PBS control) were individually added to separate tubes containing glass bubbles (a single target solution added per tube). Each tube was rotated for 1 hour, protected from light. The glass bubbles were allowed to float to the surface while tilting the tube and 160 microliters of liquid from each tube was carefully transferred to one well of a black, half area, 96-well plate. Fluorescence intensity was measured at 620 nm emission, with 590 nm excitation, and is reported in Table 6 and Figure 10. These data demonstrate that fumonisin can be extracted effectively from grains and analyzed using the methods described herein.

**TABLE 6**

| Fumonisin Concentration | % Increase in Signal (excitation 590 nm/emission 620 nm) | | | |
|---|---|---|---|---|
| | Corn | Oat | Wheat | Control |
| 100 ppb | 13,351 | 13,081 | 13,825 | 12,893 |
| 50 ppb | 13,822 | 12,891 | 13,148 | 11,648 |
| 5 ppb | 11,548 | 9,958 | 10,691 | 10,025 |
| 0 ppb | 8,796 | 7,562 | 8,354 | 7,315 |

### Preparative Example 7: Synthesis of an Aflatoxin Bl-QD 585 Conjugate

Dry DMSO (0.4 mL) was added to 50 mg of EZ-LINK hydrazide-PEG4-biotin, to make a 250 mM stock solution. A 10 microliter aliquot of this solution (2.5 micromoles) was added to 100 microliters of a 10 mg/mL solution of aflatoxin B 1 in DMSO in a brown vial. Acetic acid

(110 microliters) was added to the vial which was then heated to 50 °C overnight in a water bath. LCMS indicated consumption of the EZ-LINK hydrazide-PEG4-biotin and formation of the aflatoxin conjugate (biotin-PEG4-AFBl, MH⁺ 800.2). A 5 microliter aliquot (2 X 10⁸ mole) of this solution was diluted with 975 microliters of phosphate buffer (0. IN pH 7.6) to make a 0.02 nM solution. This solution was added to 20 microliters of a 1 micromolar solution of QDOT 585 streptavidin conjugate (obtained as part of a Q10151MP sampler kit from Thermo Fisher Scientific) and gently rotated for two hours at room temperature.

After conjugation, the solution was concentrated with two 0.5 mL AMICON Ultra centrifugal filters by centrifuging at 14,000 G for 15 minutes. The eluent (approximately 400 microliters) was removed and the concentrated solution left in the filters was washed a further three times on the filter (14,000 G, 15 minutes) with 0.4 mL of pH 7.6 buffer. The two concentrated final solutions were combined and refrigerated, yielding approximately 110 microliters of a bright orange fluorescent solution containing approximately 10¹¹ moles of QDOT 585, functionalized with aflatoxin B1 through a streptavidin-biotin-PEG4 linker.

### Proof-of-Concept Example 5: Detection of Aflatoxin by the Depletion Method with Bound DMAB2948 Antibodies

NANOSEP tubes (0.45 micron) were individually charged with 10 mg of glycidyl-functionalized XLD3000 glass bubbles (Preparative Example 2). For each tube, a 400 microliter volume of anti-aflatoxin (DMAB2948) antibody solution (approximately 0.07 mg/mL) in coupling buffer (0.1M phosphate buffer with 0.9 M sodium sulfate, pH 7.5) was added and the suspension gently rotated at 25 rpm for 1 hour. Liquid was removed by centrifugation and 0.4 mL of quenching buffer (3 M ethanolamine, pH 9) with 1% BSA was added with the contents mixed by rotation at 25 rpm for 1 hour. Liquid was removed by centrifugation and the glass bubbles were washed three times with 0.5 mL of dilution buffer (phosphate buffer, pH 7.4 with 0.1% TWEEN 20 nonionic detergent).

The antibody -functionalized glass bubbles were then suspended in 200 microliters of dilution buffer (phosphate buffer, pH 7.4 with 0.1% TWEEN 20 nonionic detergent) and 25 microliter aliquots of this suspension were carefully transferred into clean 1.5-mL tubes.

A stock solution of aflatoxin B 1 (20 micrograms/mL in methanol, Sigma- Aldrich, St Louis, MO) was diluted (PBS, 0.1% Tween-20, pH 7.4) to obtain a 1000 ppb solution. This solution was mixed with QD-PEG-AFB 1 conjugate solution from Preparative Example 7 (20 microliters) and dilution buffer in appropriate quantities to give final aflatoxin B 1 concentrations of 100 ppb, 10 ppb, 1 ppb and 0 ppb. Aliquots (175 microliters) of these solutions were individually added to separate tubes containing antibody -functionalized glass bubbles in duplicate (a single aliquot added per tube). Each tube was incubated with rotation for 30 min. The glass bubbles were allowed to float to the surface while tilting the tube and 200 microliters of liquid from each tube was carefully transferred to one well of a black, half area, 96-well plate.

Fluorescence intensity was measured at 585 nm emission, with 400 nm excitation and is reported in Table 7 and Figure 11. These data demonstrate that aflatoxin concentrations of 10 ppb and higher can be measured effectively with the depletion method.

**TABLE 7**

| Aflatoxin B1 Concentration (ppb) | Proof-of-Concept Example 5 (Fluorescence Signal Intensity) |
|---|---|
| 100 | 37,116 |
| 10 | 28,861 |
| 1 | 20,294 |
| 0 | 20,248 |

### Example 1:

PVOH (100 g) was dissolved in deionized water (233 g) by heating at 70 °C for 20 hours.

The PVOH solution was cooled to room temperature. S60HS glass bubbles (0.3 g, density of 0.6 g/mL, average diameter of 30 micrometers) were added to a bottle containing 30 g of the PVOH solution and the contents were mixed for 1 hour at 50 °C using a bottle roller mixer to provide the coating formulation.

Film samples containing immobilized glass bubbles were prepared by coating the coating formulation onto the release coated surface of a polyester release film using a notch bar coater with an 8 mil gap setting. The coated sample was dried at 65 °C for 20 minutes. Discs (2.54 cm diameter) were punched from the sample and the release film was removed. A single film disc was placed in the bottom of a vial and then 2 mL of room temperature water was added to the vial. Within 30 seconds, the film dissolved and the functionalized glass bubbles were observed to float to the upper surface of the water.

### Example 2:

The same procedure as described in Example 1 was followed with the exception that a smaller amount of S60HS glass bubbles (0.05 g) were added to the bottle containing 30 g of the PVOH solution. The resulting film disc dissolved in the water within 30 seconds and the functionalized glass bubbles were observed to float to the upper surface of the water.

### Example 3:

The same procedure as described in Example 1 was followed with the exception that 0.3 g of XLD3000 glass bubbles (density of 0.23 g/mL, average diameter of 30 micrometers) were added to the bottle containing 30 g of the PVOH solution. The resulting film disc dissolved in the water within 30 seconds and the functionalized glass bubbles were observed to float to the upper surface of the water.

## Claims

1. A composition comprising:
a film disposed on a substrate, the film comprising
a first polymer matrix material; and
a plurality of first hollow glass bubbles embedded in the first polymer matrix material;
wherein the first hollow glass bubbles have a density of less than 0.60 gram/mL, a span of less than 1.0, and a plurality of first affinity groups that are covalently attached to the first hollow glass bubbles.

2. The composition claim 1, further comprising
a plurality of second hollow glass bubbles embedded in a first water-soluble or water-degradable polymer matrix material;
wherein the second hollow glass bubbles have a density of less than 0.60 gram/mL, a span of less than 1.0, and a plurality of second affinity groups different from the first affinity groups and that are covalently attached to the first hollow glass bubbles.

3. The composition of claim 2 comprising at least two film layers,
a first film layer comprising the first water-soluble or water-degradable polymer matrix material and
a second film layer comprising a second water-soluble or water-degradable polymer matrix material; and
optionally further comprising a plurality of third hollow glass bubbles embedded in the second water-soluble or water-degradable polymer matrix material,
wherein the second hollow glass bubbles have a density of less than 0.60 gram/mL, a span of less than 1.0, and a plurality of affinity groups that are covalently attached to the first hollow glass bubbles,
wherein the plurality of affinity groups are selected from first affinity groups, second affinity groups, or combinations thereof.

4. The composition of claim 3 wherein the second water-soluble or water-degradable polymer matrix material is disposed over at least part of the first water-soluble or water-degradable polymer matrix material.

5. The composition of any of the preceding claims, wherein the first polymer matrix material comprises an extended release polymer.

6. The composition of any of claims 3-4, wherein the second water-soluble or water-degradable polymer matrix material comprises an immediate release polymer.

7. The composition of claim 6, wherein the immediate release polymer comprises hydroxypropyl methylcellulose, hydroxypropyl cellulose, hydroxyethyl cellulose, methylcellulose, sodium carboxymethyl cellulose, polyvinyl pyrrolidone, polyvinyl pyrrolidone- polyvinyl acetate copolymer, polyvinyl alcohol, copolymer of polyvinyl alcohol and polyvinyl acetate, copolymer of polyvinyl alcohol and polyethylene glycol, water-soluble (meth)acrylic polymers, water-soluble (meth)acry lie copolymers, water-soluble copolymers of (meth)acry lates and polyethylene glycol, and polyethylene glycol.

8. The composition of claim 5, wherein the extended release polymer comprises poly(glycolic acid), poly(lactic acid), poly(lactic-co-glycolic acid), poly(capralactone), poly(ortho ester), poly(anhydride), poly(amide), poly(ester amide), poly(posphoester), chitosan, hyaluronic acid, poly(alkyl cyanoacrylate), enteric polymer, or enzyme-degradable polymer.

9. The composition of any of the preceding claims, further comprising a plurality of first detector compound molecules not covalently bound to the plurality of first hollow glass bubbles,
wherein the first detector compound molecules comprises a first detectable group that is detectable at a first wavelength.

10. The composition of claim 9, further comprising a plurality of second detector compound molecules not covalently bound to the plurality of first hollow glass bubbles,
wherein said second detector compound molecules comprises a second detectable group that is detectable at a second wavelength.

11. The composition of any of the preceding claims, wherein the substrate is a vessel.

12. A method of detecting an analyte, comprising:
providing a composition according to any of claims 9-10
contacting a sample containing first free target analyte molecules with the composition;
contacting a liquid, optionally an aqueous liquid, with the composition;
allowing the first free target analyte molecules to bind to the first affinity groups or to the first detector compound molecules;
allowing the plurality of first hollow glass bubbles to float to an upper concentrated portion of the liquid; and
measuring an amount of the first detector compound molecules in a second portion of the liquid that is below the upper concentrated portion, optionally without removing the plurality of first hollow glass bubbles from the liquid.

13. The method of claim 12, wherein measuring the amount of the first detector compound molecules comprises measuring a luminescence signal at a first wavelength or measuring an absorbance at a first wavelength.

14. The method of any of-claims 12-13, further comprising:
releasing a first portion of hollow glass bubbles from a first water-soluble or water-degradable polymer matrix material within a first time period, the first time period beginning when the liquid is contacted with the composition and optionally ending when all of the first portion of hollow glass bubbles are released;
releasing a second portion of hollow glass bubbles from the first or a second water-soluble or water-degradable polymer matrix material within a second time period that is longer than the first time period, the second time period beginning when the liquid is contacted with the composition and optionally ending when all of the second portion of hollow glass bubbles are released;
wherein the first portion of hollow glass bubbles is a portion of the plurality of first hollow glass bubbles or all of the plurality of first hollow glass bubbles; and
wherein the second portion of hollow glass bubbles is a portion of the plurality of first hollow glass bubbles, a portion of the plurality of second hollow glass bubbles, or all of the plurality of second hollow glass bubbles.

15. The compostion of claim 11, wherein said vessel is selected from an Eppendorf tube, a PCR tube, a test tube, and/or a cuvette.

## Patentansprüche

1. Zusammensetzung, umfassend:
einen auf einem Substrat angeordneten Film, wobei der Film umfasst:
ein erstes Polymermatrixmaterial; und
eine Vielzahl erster hohler Glasblasen, die in das erste Polymermatrixmaterial eingebettet sind;
wobei die ersten hohlen Glasblasen eine Dichte von weniger als 0,60 g/ml, eine Spannweite von weniger als 1,0 und eine Vielzahl erster Affinitätsgruppen aufweisen, die kovalent an die ersten hohlen Glasblasen gebunden sind.

2. Zusammensetzung nach Anspruch 1, ferner umfassend:
eine Vielzahl zweiter hohler Glasblasen, die in ein erstes wasserlösliches oder wasserabbaubares Polymermatrixmaterial eingebettet sind;
wobei die zweiten hohlen Glasblasen eine Dichte von weniger als 0,60 g/ml, eine Spannweite von weniger als 1,0 und eine Vielzahl zweiter Affinitätsgruppen aufweisen, die sich von den ersten Affinitätsgruppen unterscheiden und kovalent an die ersten hohlen Glasblasen gebunden sind.

3. Zusammensetzung nach Anspruch 2, umfassend mindestens zwei Filmschichten,
wobei eine erste Filmschicht das erste wasserlösliche oder wasserabbaubare Polymermatrixmaterial umfasst und
eine zweite Filmschicht ein zweites wasserlösliches oder wasserabbaubares Polymermatrixmaterial umfasst; und
gegebenenfalls ferner umfassend eine Vielzahl dritter hohler Glasblasen, die in das zweite wasserlösliche oder wasserabbaubare Polymermatrixmaterial eingebettet sind,
wobei die zweiten hohlen Glasblasen eine Dichte von weniger als 0,60 g/ml, eine Spannweite von weniger als 1,0 und eine Vielzahl von Affinitätsgruppen aufweisen, die kovalent an die ersten hohlen Glasblasen gebunden sind, wobei die Vielzahl von Affinitätsgruppen aus ersten Affinitätsgruppen, zweiten Affinitätsgruppen oder Kombinationen davon ausgewählt ist.

4. Zusammensetzung nach Anspruch 3, wobei das zweite wasserlösliche oder wasserabbaubare Polymermatrixmaterial mindestens einen Teil des ersten wasserlöslichen oder wasserabbaubaren Polymermatrixmaterials bedeckt.

5. Zusammensetzung nach einem der vorhergehenden Ansprüche, wobei das erste Polymermatrixmaterial ein Polymer mit verlängerter Wirkstofffreisetzung umfasst.

6. Zusammensetzung nach einem der Ansprüche 3-4, wobei das zweite wasserlösliche oder wasserabbaubare Polymermatrixmaterial ein Polymer mit sofortiger Wirkstofffreisetzung umfasst.

7. Zusammensetzung nach Anspruch 6, wobei das Polymer mit sofortiger Wirkstofffreisetzung Hydroxypropylmethylcellulose, Hydroxypropylcellulose, Hydroxyethylcellulose, Methylcellulose, Natriumcarboxymethylcellulose, Polyvinylpyrrolidon, Polyvinylpyrrolidon-Polyvinylacetat-Copolymer, Polyvinylalkohol, Copolymer aus Polyvinylalkohol und Polyvinylacetat, Copolymer aus Polyvinylalkohol und Polyethylenglykol, wasserlösliche (Meth)acrylatpolymere, wasserlösliche (Meth)acrylat-Copolymere, wasserlösliche Copolymere aus (Meth)acrylaten und Polyethylenglykol sowie Polyethylenglykol umfasst.

8. Zusammensetzung nach Anspruch 5, wobei das Polymer mit verlängerter Wirkstofffreisetzung Poly(glycolsäure), Poly(milchsäure), Poly(milch-co-glycolsäure), Poly(capralacton), Poly(orthoester), Poly(anhydrid), Poly(amid), Poly(esteramid), Poly(posphoester), Chitosan, Hyaluronsäure, Poly(alkylcyanoacrylat), magensaftresistentes Polymer oder enzymabbaubares Polymer umfasst.

9. Zusammensetzung nach einem der vorhergehenden Ansprüche, ferner umfassend eine Vielzahl von Molekülen einer ersten Detektorverbindung, die nicht kovalent an die Vielzahl der ersten hohlen Glasblasen gebunden sind,
wobei die Moleküle der ersten Detektorverbindung eine erste detektierbare Gruppe aufweisen, die bei einer ersten Wellenlänge detektierbar ist.

10. Zusammensetzung nach Anspruch 9, ferner umfassend eine Vielzahl von Molekülen einer zweiten Detektorverbindung, die nicht kovalent an die Vielzahl der ersten hohlen Glasblasen gebunden sind,
wobei die Moleküle der zweiten Detektorverbindung eine zweite detektierbare Gruppe aufweisen, die bei einer zweiten Wellenlänge detektierbar ist.

11. Zusammensetzung nach einem der vorhergehenden Ansprüche, wobei das Substrat ein Gefäß ist.

12. Verfahren zum Nachweis eines Analyten, umfassend:
das Bereitstellen einer Zusammensetzung nach einem der Ansprüche 9-10;
das Inkontaktbringen einer Probe, die erste freie Zielanalytmoleküle enthält, mit der Zusammensetzung;
Inkontaktbringen einer Flüssigkeit, gegebenenfalls einer wässrigen Flüssigkeit, mit der Zusammensetzung;
das Ermöglichen der Bindung der ersten freien Zielanalytmoleküle an die ersten Affinitätsgruppen oder an die Moleküle der ersten Detektorverbindung;
das Aufsteigenlassen der Vielzahl der ersten hohlen Glasblasen in einen oberen, konzentrierten Bereich der Flüssigkeit; und
das Messen der Menge der Moleküle der ersten Detektorverbindung in einem zweiten, unterhalb des oberen, konzentrierten Bereichs liegenden Bereich der Flüssigkeit, gegebenenfalls ohne die Vielzahl der ersten hohlen Glasblasen aus der Flüssigkeit zu entfernen.

13. Verfahren nach Anspruch 12, wobei das Messen der Menge der Moleküle der ersten Detektorverbindung das Messen eines Lumineszenzsignals bei einer ersten Wellenlänge oder das Messen der Absorption bei einer ersten Wellenlänge umfasst.

14. Verfahren nach einem der Ansprüche 12-13, ferner umfassend:
das Freisetzen eines ersten Anteils an hohlen Glasblasen aus einem ersten wasserlöslichen oder wasserabbaubaren Polymermatrixmaterial innerhalb eines ersten Zeitraums, wobei der erste Zeitraum beginnt, wenn die Flüssigkeit mit der Zusammensetzung in Kontakt gebracht wird, und gegebenenfalls endet, wenn der gesamte erste Anteil an hohlen Glasblasen freigesetzt ist;
das Freisetzen eines zweiten Anteils an hohlen Glasblasen aus dem ersten oder einem zweiten wasserlöslichen oder wasserabbaubaren Polymermatrixmaterial innerhalb eines zweiten Zeitraums, der länger als der erste Zeitraum ist, wobei der zweite Zeitraum beginnt, wenn die Flüssigkeit mit der Zusammensetzung in Kontakt gebracht wird, und gegebenenfalls endet, wenn der gesamte zweite Anteil an hohlen Glasblasen freigesetzt ist;
wobei der erste Anteil an hohlen Glasblasen ein Anteil der Vielzahl der ersten hohlen Glasblasen oder die gesamte Vielzahl der ersten hohlen Glasblasen ist; und
wobei der zweite Anteil an hohlen Glasblasen ein Anteil der Vielzahl der ersten hohlen Glasblasen, ein Anteil der Vielzahl der zweiten hohlen Glasblasen oder die gesamte Vielzahl der zweiten hohlen Glasblasen ist.

15. Zusammensetzung nach Anspruch 11, wobei das Gefäß aus einem Eppendorf-Gefäß, einem PCR-Röhrchen, einem Reagenzglas und/oder einer Küvette ausgewählt ist.

## Revendications

1. Composition comportant :
un film disposé sur un substrat, le film comportant :
un premier matériau de matrice polymère ; et
une pluralité de premières bulles de verre creuses incorporées dans le premier matériau de matrice polymère ;
dans laquelle les premières bulles de verre creuses ont une masse volumique inférieure à 0,60 g/ml, une étendue inférieure à 1,0, et une pluralité de premiers groupes d'affinité qui sont liés par covalence aux premières bulles de verre creuses.

2. Composition selon la revendication 1, comportant en outre :
une pluralité de deuxièmes bulles de verre creuses incorporées dans un premier matériau de matrice polymère soluble dans l'eau ou dégradable dans l'eau ;
dans laquelle les deuxièmes bulles de verre creuses ont une masse volumique inférieure à 0,60 g/ml, une étendue inférieure à 1,0, et une pluralité de seconds groupes d'affinité différents des premiers groupes d'affinité et qui sont liés par covalence aux premières bulles de verre creuses.

3. Composition selon la revendication 2, comportant au moins deux couches de film,
une première couche de film comportant le premier matériau de matrice polymère soluble dans l'eau ou dégradable dans l'eau et
une seconde couche de film comportant un second matériau de matrice polymère soluble dans l'eau ou dégradable dans l'eau ; et
comportant facultativement en outre une pluralité de troisièmes bulles de verre creuses incorporées dans le second matériau de matrice polymère soluble dans l'eau ou dégradable dans l'eau,
dans laquelle les deuxièmes bulles de verre creuses ont une masse volumique inférieure à 0,60 g/ml, une étendue inférieure à 1,0, et une pluralité de groupes d'affinité qui sont liés par covalence aux premières bulles de verre creuses,
dans laquelle les groupes de la pluralité de groupes d'affinité sont choisis parmi des premiers groupes d'affinité, des seconds groupes d'affinité ou des combinaisons de ceux-ci.

4. Composition selon la revendication 3, dans laquelle le second matériau de matrice polymère soluble dans l'eau ou dégradable dans l'eau est disposé sur au moins une partie du premier matériau de matrice polymère soluble dans l'eau ou dégradable dans l'eau.

5. Composition selon l'une quelconque des revendications précédentes, dans laquelle le premier matériau de matrice polymère comporte un polymère à libération prolongée.

6. Composition selon l'une quelconque des revendications 3 à 4, dans laquelle le second matériau de matrice polymère soluble dans l'eau ou dégradable dans l'eau comporte un polymère à libération immédiate.

7. Composition selon la revendication 6, dans laquelle le polymère à libération immédiate comporte de l'hydroxypropyl méthylcellulose, de l'hydroxypropyl cellulose, de l'hydroxyéthyl cellulose, de la méthylcellulose, de la carboxyméthyl cellulose sodique, de la polyvinyl pyrrolidone, un - copolymère de polyvinyl pyrrolidone et d'acétate de polyvinyle, de l'alcool polyvinylique, un copolymère d'alcool polyvinylique et d'acétate de polyvinyle, un copolymère d'alcool polyvinylique et de polyéthylène glycol, des polymères (méth)acryliques solubles dans l'eau, des copolymères (méth)acryliques solubles dans l'eau, des copolymères de (méth)acrylates et de polyéthylène glycol solubles dans l'eau, et du polyéthylène glycol.

8. Composition selon la revendication 5, dans laquelle le polymère à libération prolongée comporte un poly(acide glycolique), un poly(acide lactique), un poly(acide lactique-co-glycolique), une poly(capralactone), un poly(ortho ester), un poly(anhydride), un poly(amide), un poly(ester amide), un poly(phosphoester), du chitosane, de l'acide hyaluronique, un poly(cyanoacrylate d'alkyle), un polymère entérique ou un polymère dégradable par enzyme.

9. Composition selon l'une quelconque des revendications précédentes, comportant en outre une pluralité de premières molécules de composé détecteur non liées par covalence à la pluralité de premières bulles de verre creuses,
dans laquelle les premières molécules de composé détecteur comportent un premier groupe détectable qui est détectable à une première longueur d'onde.

10. Composition selon la revendication 9, comportant en outre une pluralité de secondes molécules de composé détecteur non liées par covalence à la pluralité de premières bulles de verre creuses,
dans laquelle lesdites secondes molécules de composé détecteur comportent un second groupe détectable qui est détectable à une seconde longueur d'onde.

11. Composition selon l'une quelconque des revendications précédentes, dans laquelle le substrat est un récipient.

12. Procédé de détection d'un analyte, comportant de :
fournir une composition selon l'une quelconque des revendications 9 à 10 ;
mettre en contact un échantillon contenant des premières molécules d'analyte cibles libres avec la composition ;
mettre en contact un liquide, facultativement un liquide aqueux, avec la composition ;
laisser les premières molécules d'analyte cibles libres se lier aux premiers groupes d'affinité ou aux premières molécules de composé détecteur ;
laisser la pluralité de premières bulles de verre creuses flotter jusqu'à une portion concentrée supérieure du liquide ; et
mesurer une quantité des premières molécules de composé détecteur dans une seconde portion du liquide qui se situe en dessous de la portion concentrée supérieure, facultativement sans retirer la pluralité de premières bulles de verre creuses du liquide.

13. Procédé selon la revendication 12, dans lequel la mesure de la quantité des premières molécules de composé détecteur comporte de mesurer un signal de luminescence à une première longueur d'onde ou de mesurer une absorbance à une première longueur d'onde.

14. Procédé selon l'une quelconque des revendications 12 à 13, comportant en outre de :
libérer une première portion de bulles de verre creuses à partir d'un premier matériau de matrice polymère soluble dans l'eau ou dégradable dans l'eau sur une première période de temps, la première période de temps débutant lorsque le liquide est mis en contact avec la composition, et se terminant facultativement lorsque les bulles de la première portion de bulles de verre creuses sont toutes libérées ;
libérer une seconde portion de bulles de verre creuses à partir du premier ou d'un second matériau de matrice polymère soluble dans l'eau ou dégradable dans l'eau sur une seconde période de temps qui est plus longue que la première période de temps, la seconde période de temps débutant lorsque le liquide est mis en contact avec la composition et se terminant facultativement lorsque les bulles de la seconde portion de bulles de verre creuses sont toutes libérées ;
dans lequel la première portion de bulles de verre creuses est une portion de la pluralité de premières bulles de verre creuses ou la totalité de la pluralité de premières bulles de verre creuses ; et
dans lequel la seconde portion de bulles de verre creuses est une portion de la pluralité de premières bulles de verre creuses, une portion de la pluralité de secondes bulles de verre creuses, ou la totalité de la pluralité de secondes bulles de verre creuses.

15. Composition selon la revendication 11, dans laquelle ledit récipient est choisi parmi un tube Eppendorf, un tube de PCR, un tube à essai et/ou une cuvette.
